# EUROPÄISCHE PATENTSCHRIFT

(11) **EP 2 897 927 B1**
(45) Veröffentlichungstag und Bekanntmachung des Hinweises auf die Patenterteilung: **22.06.2016**
(21) Anmeldenummer: 13763254.3
(22) Anmeldetag: 18.09.2013
(51) Int. Cl.: C07C 5/333, C07C 5/48, B01J 35/04, C10G 70/00, C07C 7/148, B01J 23/63, C01B 3/26, B01J 23/62, C07C 11/08, C07C 11/167

(54) **VERFAHREN ZUR HERSTELLUNG VON BUTADIEN MIT ENTFERNUNG VON SAUERSTOFF AUS C4-KOHLENWASSERSTOFFSTRÖMEN**
METHOD FOR PRODUCING BUTADIENE WITH REMOVAL OF OXYGEN FROM C4 HYDROCARBON STREAMS
PROCÉDÉ DE PRODUCTION DE BUTADIÈNE PAR ÉLIMINATION DE L'OXYGÈNE DE FLUX D'HYDROCARBURES EN C4

(30) Priorität: 20.09.2012 EP 12185150
(43) Veröffentlichungstag der Anmeldung: 29.07.2015
(73) Patentinhaber: BASF SE, 67056 Ludwigshafen am Rhein (DE)
(72) Erfinder: REZAI, Alireza, 68163 Mannheim (DE); AVERLANT, Gauthier Luc Maurice, 60323 Frankfurt (DE); DIETERLE, Martin, 67059 Ludwigshafen (DE); MABANDE, Godwin, Novi, MI 48375 (US)
(86) Internationale Anmeldenummer: PCT/EP2013/069329
(87) Internationale Veröffentlichungsnummer: WO 2014/044693

(56) Entgegenhaltungen:
- WO-A1-2006/075025
- WO-A1-2010/133565

## Beschreibung

Die Erfindung betrifft ein Verfahren zur Herstellung von Butadien mit Entfernung von Sauerstoff aus freien Sauerstoff enthaltenden C₄-Kohlenwasserstoffströmen.

Bei der Herstellung von Butadien aus Butan können freien Sauerstoff enthaltende C₄-Kohlenwasserstoffströme anfallen, aus denen der freie Sauerstoff entfernt werden soll oder muss, da er zur Bildung von Peroxiden führen kann, die unter Sicherheitsaspekten schwierig zu handhaben sind.

Die WO 2006/075025 beschreibt ein Verfahren zur Herstellung von Butadien aus n-Butan durch nicht-oxidative, katalytische Dehydrierung von n-Butan, nachfolgende oxidative Dehydrierung und Aufarbeitung des Produktgemisches. Nach der oxidativen Dehydrierung kann der im Produktgasstrom verbliebene Sauerstoff entfernt werden, beispielsweise indem er katalytisch mit Wasserstoff umgesetzt wird. Ein entsprechender C₄-Produktgasstrom kann dabei 20 bis 80 Vol.-% Butadien, 20 bis 80 Vol.-% n-Butan, 0,5 bis 50 Vol.-% 2-Buten und 0 bis 20 Vol.-% 1-Buten sowie geringe Mengen Sauerstoff enthalten. Der Rest-Sauerstoff kann sich insoweit als störend auswirken, als er in nachgelagerten Verfahrensschritten als Initiator für Polymerisationsreaktionen wirken kann. Diese Gefahr ist insbesondere bei der destillativen Abtrennung von Butadien gegeben und kann dort zu Ablagerungen von Polymeren (Bildung von so genanntem "Popcorn") in der Extraktiv-Destillationskolonne führen. Deshalb wird eine SauerstoffEntfernung unmittelbar nach der oxidativen Dehydrierung durchgeführt, im Allgemeinen durch eine katalytische Verbrennungsstufe, in der Sauerstoff mit dem im Gasstrom enthaltenen Wasserstoff in Gegenwart eines Katalysators umgesetzt wird. Hierdurch wird eine Verringerung des Sauerstoffgehalts bis auf geringe Spuren erreicht. Als geeigneter Katalysator wird α-Aluminiumoxid beschrieben, das 0,01 bis 0,1 Gew.-% Platin und 0,01 bis 0,1 Gew.-% Zinn enthält. Alternativ werden auch Kupfer in reduzierter Form enthaltende Katalysatoren angegeben.

Die WO 2010/130610 beschreibt ein Verfahren zur Herstellung von Propylenoxid durch Umsetzung von Propen mit Wasserstoffperoxid, Abtrennung des Propylenoxids unter Erhalt eines Propen und Sauerstoff enthaltenden Gasgemisches. Diesem Gasgemisch wird Wasserstoff zugesetzt, und der enthaltene Sauerstoff wird zumindest teilweise durch Umsetzung mit dem Wasserstoff in Gegenwart eines kupferhaltigen Katalysators umgesetzt. Dabei enthält der Katalysator 30 bis 80 Gew.-% Kupfer, berechnet als CuO.

Die WO 2010/113565 betrifft einen Monolithkatalysator und seine Verwendung. Ein Monolithkatalysator, der Pt, Sn, K, Cs und La auf einem SiO₂/ZrO₂-Mischoxid als Träger enthält, wird für die Dehydrierung von Alkanen zu Alkenen, insbesondere von Propan zu Propen oder n-Butan zu Butenen oder für die katalytische Verbrennung von Wasserstoff mit Sauerstoff eingesetzt.

Neben der "Popcorn"-Bildung können in kohlenwasserstoffhaltigen Gasgemischen, insbesondere Butadien und Sauerstoff enthaltenden Gasgemischen, der Sauerstoffgehalt zur Desaktivierung von Katalysatoren, zu Rußablagerungen, Peroxid-Bildungen, zur Verschlechterung der Adsorptionseigenschaften von Lösungsmitteln im Aufarbeitungsprozess beitragen.

WO 2012/117085 offenbart die Verwendung von N-Methylpyrrolidon als Absorptions- und Extraktionslösungsmittel zu einem Verfahren gemäß WO 2006/075025.

Insbesondere bei der Herstellung von Butadien aus n-Butan bildet die selektive Sauerstoffentfernung eine Grundvoraussetzung für die wirtschaftliche Durchführbarkeit des Verfahrens, da jeder Verlust des Zielproduktes Butadien mit höheren Kosten verbunden ist. Die zu erfüllende Spezifikation liegt bei einer Rest-Sauerstoff-Konzentration nach der Sauerstoffentfernungsstufe von weniger als 100 ppm.

Aufgabe der vorliegenden Erfindung ist die Bereitstellung verbesserter Verfahren für die katalytische Sauerstoff-Abtrennung aus C₄-Kohlenwasserstoff-Gemischen. Der Katalysator soll es erlauben, bei einem Gehalt an freiem Wasserstoff im Kohlenwasserstoffstrom die selektive Umsetzung von freiem Sauerstoff mit freiem Wasserstoff zu katalysieren, ohne dass dabei nennenswerte Mengen an C₄-Kohlenwasserstoffen, insbesondere Butadien, mit umgesetzt werden.

Die Aufgabe wird erfindungsgemäß gelöst durch ein Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Butene, Butadien, Wasserstoff, gegebenenfalls Wasserdampf, gegebenenfalls Kohlenstoffoxide und gegebenenfalls Inertgase erhalten wird;
G) Einspeisung eines aus dem Gasstrom b gewonnenen Butan, Butene, Butadien enthaltenden Stoffstroms f und eines sauerstoffhaltigen Gases in mindestens eine zweite Dehydrierzone und oxidative Dehydrierung von 1-Buten und 2-Butenen, wobei ein Gasstrom g enthaltend n-Butan, nicht umgesetzte(s) 1-Buten und 2-Butene, Butadien, Wasserdampf, gegebenenfalls Kohlenstoffoxide, gegebenenfalls Wasserstoff und gegebenenfalls Inertgase erhalten wird, und
H) Entfernung des in dem Gasstrom g enthaltenen Restsauerstoffs mittels katalytischer Verbrennungsstufe, in der der Sauerstoff mit einem Teil des oder dem gesamten zuvor abgetrennten Wasserstoff(s) d2 und/oder zusätzlich eingespeistem Wasserstoff umgesetzt wird unter Erhalt eines sauerstoffabgereicherten Stoffstromes h,
dadurch gekennzeichnet, dass Stufe H) in Gegenwart eines Katalysators durchgeführt wird, der einen Monolithen umfasst, der ein katalytisch inertes Material mit einer niedrigen BET-Oberfläche von weniger als 10 m²/g und einer Katalysatorschicht aufweist, die auf den Monolithen aufgebracht wurde und ein oxidisches Trägermaterial, mindestens ein Edelmetall ausgewählt aus der Gruppe bestehend aus den Edelmetallen der Gruppe VIII des Periodensystems der Elemente, gegebenenfalls Zinn und/oder Rhenium, und gegebenenfalls weitere Metalle enthält, wobei die Dicke der Katalysatorschicht 5 bis 500 µm beträgt, und das Verfahren zwischen den Stufen B) und G) die nachfolgenden Stufen C) bis F) umfasst, wobei der Stoffstrom f in Stufe G) geführt wird:
C) Kompression in mindestens einer ersten Kompressionsstufe und Abkühlung des Gasstroms b, wobei mindestens ein Kondensatstrom c1 enthaltend Wasser und ein Stoffstrom c2 enthaltend Butene und Butadien, n-Butan, Wasserstoff, Wasserdampf, gegebenenfalls Kohlenstoffoxide und gegebenenfalls Inertgase erhalten wird;
D) Absorption der Butene und des Butadien, n-Butan, Wasserstoff, Wasserdampf, gegebenenfalls Inertgase und gegebenenfalls Kohlenstoffoxide enthaltenden Stoffstroms c2 mit einem 80 bis 97 Gew.-% N-Methylpyrrolidon und 3 bis 20 Gew.-% Wasser enthaltenen Gemisch als selektivem Lösungsmittel, wobei ein N-Methylpyrrolidon, Wasser sowie Butene, Butadien, Butan und gegebenenfalls Kohlendioxid enthaltender Stoffstrom d1 und ein Wasserstoff und gegebenenfalls Inertgase und Butan enthaltender Stoffstrom d2 erhalten werden;
E) Extraktivdestillation des N-Methylpyrrolidon, Wasser sowie Butene, Butadien, Butan und gegebenenfalls Kohlenstoffoxide enthaltenden Stoffstrom d1 mit einem 80 bis 97 Gew.-% N-Methylpyrrolidon und 3 bis 20 Gew.-% Wasser enthaltenden Stoffstrom e1 als selektivem Lösungsmittel, wobei der N-Methylpyrrolidon, Wasser sowie Butene, Butadien, Butan und gegebenenfalls Kohlenstoffoxide enthaltende Stoffstrom d1 in einen N-Methylpyrrolidon, Wasser sowie Butan, Butene, Butadien enthaltenden Stoffstrom e2 sowie einen im Wesentlichen Butan und gegebenenfalls Kohlenstoffoxide enthaltenden Stoffstrom e3 getrennt wird;
F) Destillation des N-Methylpyrrolidon, Wasser, Butan sowie Butene, Butadien enthaltenden Stoffstroms e2 in einen im Wesentlichen N-Methylpyrrolidon und Wasser enthaltenden Stoffstrom e1 und einen Butan, Butene, Butadien enthaltenden Stoffstrom f.

Zunächst wird der in Stufe H) eingesetzte Katalysator näher erläutert.

Der Katalysator umfasst vorzugsweise Platin und Zinn.

Vorzugsweise enthält die Katalysatorschicht ein Metall aus der dritten Übergangsgruppe des Periodensystems der Elemente einschließlich der Lanthanide, insbesondere Lanthan.

Vorzugsweise enthält die Katalysatorschicht mindestens ein Alkalimetall oder Erdalkalimetall, besonders bevorzugt Kalium und/oder Cäsium.

Das oxidische Trägermaterial ist vorzugsweise ausgewählt aus Oxiden der Metalle der zweiten, dritten und vierten Hauptgruppe und der dritten und vierten Übergangsgruppe. Besonders bevorzugt ist das Trägermaterial ausgewählt aus Oxiden von Magnesium, Calcium, Aluminium, Silicium, Titan und Zirconium oder Gemischen davon, insbesondere aus Siliciumdioxid (SiO₂) und/oder Zircondioxid (ZrO₂).

Der Monolith ist vorzugsweise aus Cordierit aufgebaut.

Der in Stufe H) eingesetzte Katalysator ist an sich bekannt und beispielsweise in der WO 2010/133565 beschrieben. Für den genauen Aufbau des Katalysators sowie seine Herstellung kann auf diese Schrift verwiesen werden.

Der eingesetzte Festbettkatalysator weist einen signifikant verminderten Edelmetallgehalt und eine verbesserte Leistungsfähigkeit auf. Die Eindringtiefe des Katalysators ist auf 5 bis 500 µm, vorzugsweise 5 bis 250 µm, insbesondere 25 bis 250 µm, speziell 50 bis 250 µm beschränkt. Die Eindringtiefe des Katalysators ist durch die auf den Monolithen aufgebrachte Katalysatorschicht limitiert.

Die Katalysatorschicht auf dem Monolithen umfasst mindestens ein keramisches Oxid als Katalysatorträger und mindestens ein Edelmetall, ausgewählt aus den Elementen der Übergangsgruppen VIII des Periodensystems der Elemente, speziell Palladium, Platin oder Rhodium, gegebenenfalls Rhenium und/oder Zinn. Ein Katalysatorträger ist aus einem oder mehreren keramischen Oxiden von Elementen der zweiten, dritten und vierten Hauptgruppe und der dritten und vierten Übergangsgruppe (Gruppe IV B) des Periodensystems der Elemente und den Lanthaniden aufgebaut, speziell MgO, CaO, Al₂O₃, SiO₂, ZrO₂, TiO₂, La₂O₃ und Ce₂O₃. In einer besonders bevorzugten Ausführungsform umfasst der Katalysatorträger SiO₂ und ZrO₂, es handelt sich insbesondere um ein gemischtes Oxid von SiO₂ und ZrO₂.

Zusätzlich zu den Edelmetallen der Übergangsgruppe VIII ist es möglich, weitere Elemente in der katalytisch aktiven Schicht einzusetzen, beispielsweise Rhenium und/oder Zinn. Ferner kann eine Dotierung mit Verbindungen der dritten Hauptgruppe oder Nebengruppe (III A oder III B) oder basischen Verbindungen, wie Alkalimetallen, Erdalkalimetallen oder seltenen Erden oder deren Verbindungen erfolgen, die in die entsprechende Oxide bei Temperaturen oberhalb von 400 °C umgewandelt werden können. Die simultane Dotierung mit einer Mehrzahl der genannten Elemente oder deren Verbindungen ist möglich. Geeignete Beispiele sind Kalium und Lanthanverbindungen. Zudem kann der Katalysator mit Schwefel, Tellur, Arsen, Antimon oder Selen oder deren Verbindungen vermischt werden, die in vielen Fällen zu einem Anstieg der Selektivität führen.

Die Katalysatorschicht umfasst mindestens ein Edelmetall aus der Gruppe VIII des Periodensystems der Elemente (Ru, Rh, Pd, Os, Ir, Pt). Das bevorzugte Edelmetall ist Platin. Die Katalysatorschicht kann gegebenenfalls Zinn und/oder Rhenium enthalten. Sie enthält vorzugsweise Zinn.

In einer bevorzugten Ausführungsform enthält die Katalysatorschicht Platin und Zinn.

Zudem kann die Katalysatorschicht mit weiteren Metallen dotiert sein, beispielsweise mit Metallen der dritten Übergangsgruppe (Gruppe III B) des Periodensystems der Elemente, einschließlich der Lanthaniden (Sc, Y, La, Ce, Pr, Nd, Sm, Eu, Gd, Tb, Dy, Ho, Er, Tm, Yb, Lu). Bevorzugt werden Cer und Lanthan eingesetzt, insbesondere Lanthan.

Zudem kann die Katalysatorschicht Metalle aus den Metallen der Hauptgruppen I und II des Periodensystems der Elemente enthalten. Vorzugsweise umfasst die Katalysatorschicht Kalium und/oder Cäsium.

Speziell bevorzugt ist eine Katalysatorschicht, die Platin, Zinn, Lanthan, Kalium und Cäsium aufweist.

Die Katalysatorschicht wird auf den Monolithen durch Washcoating aufgebracht. Es ist auch möglich, zunächst die Katalysatorträgerschicht aus dem oxidischen Trägermaterial auf den Monolithen durch Washcoating aufzubringen und nachfolgend diese Schicht mit einer oder mehreren unterschiedlichen Lösungen der Metalle oder Metallverbindungen zu imprägnieren.

Geeignete Monolithstrukturen können metallisch oder keramisch sein. Sie sind vorzugsweise aus einzelnen Blöcken mit kleinen (0,5 bis 4 mm Durchmesser) parallelen Kanälen aufgebaut. Besonders bevorzugt wird Cordierit als Material für die monolithischen Strukturen eingesetzt. Entsprechende Monolithen weisen eine niedrige BET-Oberfläche auf, im Falle von Cordierit beispielsweise 0,7 m²/g. Eine niedrige BET-Oberfläche ist im Rahmen der Erfindung eine BET-Oberfläche von weniger als 10 m²/g.

Für eine genauere Beschreibung der Cordierit-Monolithe kann auf WO 2010/133565, Seiten 4 bis 6 verwiesen werden.

Die monolithische Struktur wird mit einer Katalysatorträgerschicht (Washcoat mindestens eines keramischen Oxids) oder einer Katalysatorschicht, die die katalytisch aktiven Metalle auf der keramischen Oxidträgerschicht umfasst, beschichtet. Für das Washcoating-Verfahren kann ebenfalls auf WO 2010/133565, insbesondere Seiten 6 bis 12 verwiesen werden.

Im Katalysator kann der Anteil an Elementen der Übergangsgruppe VIII und gegebenenfalls Rhenium oder Zinn im Bereich von 0,005 bis 5 Gew.-%, vorzugsweise 0,1 bis 2 Gew.-%, insbesondere 0,05 bis 1,5 Gew.-% liegen. Wenn Rhenium oder Zinn zusätzlich verwendet werden, kann ihr Mengenverhältnis zum Edelmetall im Bereich von 0,1 : 1 bis 20 : 1, vorzugsweise 1 : 1 bis 10 : 1 liegen.

Das Katalysatormaterial hat üblicherweise eine BET-Oberfläche von bis zu 500 m²/g, vorzugsweise 2 bis 300 m²/g, insbesondere 5 bis 300 m²/g. Das Porenvolumen beträgt üblicherweise zwischen 0,1 und 1 ml/g, vorzugsweise 0,15 bis 0,6 ml/g, insbesondere 0,2 bis 0,4 ml/g. Der mittlere Porendurchmesser der Mesoporen, bestimmt durch Quecksilberpenetrationsanalyse, beträgt im Allgemeinen 8 bis 60 nm, vorzugsweise 10 bis 50 nm.

Der Anteil an Poren mit einer Porengröße von mehr als 20 nm liegt üblicherweise in einem Bereich von 0 bis 90 %.

Für die Herstellung der katalytisch aktiven Schicht einschließlich Katalysatorträger kann wiederum auf WO 2010/133565, insbesondere Seiten 12 bis 18 verwiesen werden. Die Menge an Edelmetall in den Katalysatoren beträgt vorzugsweise 0,005 bis 1 Gew.-%, insbesondere 0,05 bis 0,5 Gew.-%.

Der erfindungsgemäß eingesetzte Katalysator der Stufe H) hat dabei den Vorteil, dass er insbesondere die Umsetzung von Wasserstoff mit Sauerstoff katalysiert, ohne dass es dabei zu einer nennenswerten Umsetzung von C₄-Kohlenwasserstoff, insbesondere Butadien, mit dem freien Sauerstoff kommt.

In dem erfindungsgemäßen Verfahren werden zwischen den Stufen B) und G) die Stufen C) bis F) durchgeführt, wobei der Stoffstrom f in Stufe G) geführt wird:

Vorzugsweise wird der Stoffstrom d2 ganz oder teilweise in die erste Dehydierzone B) zurückgeführt.

Dabei kann in Stufe G) ein zusätzlicher Feedstrom eingespeist werden.

Vorzugsweise wird der Stoffstrom e1 ganz oder teilweise in die Absorptionszone D) und die Extraktionsdestillationszone E) zurückgeführt.

Vorzugsweise wird der Stoffstrom e3 ganz oder teilweise in Stufe A) zurückgeführt.

Kohlenstoffoxide sind Kohlendioxid, Kohlenmonoxid oder Gemische davon.

Vorzugsweise werden nach H) die folgenden Stufen I) bis L) durchgeführt:
I) Kompression in mindestens einer ersten Kompressionsstufe und Abkühlung des sauerstoffabgereicherten Stoffstromes h oder Gasstroms g, wobei mindestens ein Kondensatstrom i1 enthaltend Wasser und ein Gasstrom i2 enthaltend n-Butan, 1-Buten, 2-Butene, Butadien, Wasserstoff, Wasserdampf, gegebenenfalls Kohlenstoffoxide und gegebenenfalls Inertgase erhalten wird;
J) Abtrennung der nicht kondensierbaren und leicht siedenden Gasbestandteile, umfassend Wasserstoff, Sauerstoff, Kohlenstoffoxide, die leicht siedenden Kohlenwasserstoffe Methan, Ethan, Ethen, Propan, Propen und Inertgase als Gasstrom j2 aus dem Gasstrom i2, wobei ein C₄-Produktgasstrom j1 erhalten wird, der im Wesentlichen aus C₄-Kohlenwasserstoffen besteht;
K) Auftrennung des Gasstroms j1 durch Extraktivdestillation mit einem selektiven Lösungsmittel, vorzugsweise einem 80 bis 97 Gew.-% N-Methylpyrrolidon und 3 bis 20 Gew.-% Wasser enthaltendem Gemisch in einen im Wesentlichen aus Butadien und selektivem Lösungsmittel, vorzugsweise N-Methylpyrrolidon bestehenden oder diese enthaltenden Stoffstrom k1 und einen n-Butan, Butene, Wasserdampf und gegebenenfalls Inertgase enthaltenden Stoffstrom k2.
L) Destillation des selektiven Lösungsmittels, vorzugsweise N-Methylpyrrolidon, Wasser und Butadien enthaltenden Stoffstroms k1 in einem im Wesentlichen selektiven Lösungsmittel, vorzugsweise N-Methylpyrrolidon und Wasser enthaltenden Stoffstrom I1 und einen Butadien enthaltenden Stoffstrom 12.

Vorzugsweise wird nach Stufe L) die folgende Stufe M) durchgeführt:
M) Reindestillation des Butadien enthaltenen Stoffstromes I2 in einer oder zwei Kolonnen, bei der ein Butadien enthaltender Strom m2 gewonnen wird und ein gegenüber Butadien leichter flüchtige Verunreinigungen enthaltender Gasstrom m1 und/oder ein gegenüber Butadien schwerer flüchtige Verunreinigungen enthaltender Sumpfstrom m3 abgetrennt werden.

Vorzugsweise wird der Gasstrom j2 ganz oder teilweise in die zweite Dehydrierzone G) zurückgeführt.

Vorzugsweise wird der Stoffstrom k2 ganz oder teilweise in den Einsatzgasstrom in Stufe A), die Absorptionsstufe D), die Extraktionsstufe E) und/oder teilweise die zweite Dehydrierzone G) zurückgeführt.

Vorzugsweise wird die Abtrennung in Stufe J) zweistufig durch Absorption mit nachfolgender Desorption durchgeführt.

Vorzugsweise wird der Stoffstrom I1 ganz oder teilweise in die Stufe K) zurückgeführt.

Vorzugsweise wird die nicht-oxidative, katalytische Dehydrierung von n-Butan autotherm unter Einspeisung eines sauerstoffhaltigen Gases durchgeführt. Das sauerstoffhaltige Gas kann dabei beispielsweise Luft, mit Sauerstoff angereicherte Luft oder technisch reiner Sauerstoff sein.

Der n-Butan enthaltene Einsatzstrom a kann dabei aus liquefied petroleum gas (LPG) gewonnen sein.

Das erfindungsgemäße Verfahren erlaubt vorzugsweise eine optimale Nutzung des eingesetzten Butans und einen optimierten Betrieb einer zweiten Dehydrierstufe durch die angegebene Aufarbeitung nach der ersten Dehydrierstufe.

Für die vorliegende Trennaufgabe sind selektive Lösungsmittel geeignet, deren Affinität zu C₄-Kohlenwasserstoffen mit Einfachbindungen in Richtung zu C₄-Kohlenwasserstoffen mit Doppelbindungen und weiter zu konjugierten Doppelbindungen und Dreifachbindungen zunimmt.

Aus apparatetechnischen Gründen werden wenig oder nicht korrosive Substanzen bevorzugt.

Erfindungsgemäß werden sowohl als Lösungsmittel für die Absorption in Schritt D) als auch als Extraktionsmittel für die Extraktion in Schritt E) und in Schritt K) eine Mischung aus 80 bis 97 Gew.-% N-Methylpyrrolidon und 3 bis 20 Gew.-% Wasser, bevorzugt eine Mischung aus 90 bis 93 Gew.-% N-Methylpyrrolidon und 7 bis 10 Gew.-% Wasser und insbesondere eine Mischung aus 91 bis 92 Gew.-% N-Methylpyrrolidon und 8 bis 9 Gew.% Wasser, beispielsweise eine Mischung aus 91,7 Gew.-% N-Methylpyrrolidon und 8,3 Gew.-% Wasser eingesetzt.

Das Verfahren ist im Vergleich zur Erzeugung von Butadien durch Steamcracken durch eine hohe Selektivität gekennzeichnet. Es fallen keine unerwünschten Koppelprodukte an. Es entfällt die aufwendige Abtrennung von Butadien aus dem Produktgasgemisch des Crackprozesses.

Das erfindungsgemäße Verfahren zeichnet sich durch eine besonders effektive Ausnutzung der Rohstoffe aus. So werden Verluste des Rohstoffs n-Butan durch bevorzugte Rückführung von nicht umgesetztem n-Butan aus der Verfahrensstufe E) in die erste Dehydrierungsstufe minimiert. Das n-Butan wird nicht (vollständig) durch die Verfahrensschritte F) - J) geführt, wodurch diese Apparate kleiner ausgeführt werden können.

Durch die bevorzugte Auftrennung von Buten und Butan nach der ersten Dehydrierstufe und bevorzugte Rückführung des Butans wird ein höherer Umsatz von Butan zu Buten erzielt, als bei Rückführung eines Butan/Butengemisches aus der Extraktvidestillation nach der zweiten Dehydrierstufe. Nicht umgesetztes Buten wird bevorzugt in die zweite Dehydrierungsstufe rückgeführt. Die teilweise Abtrennung eines butenhaltigem Produktstromes mit durch die Extraktivdestillation einstellbarem Butengehalt ist hier möglich.

Es ist außerdem möglich, in die oxidative Dehydrierungsstufe G) einen butenhaltigen C₄-Strom zusätzlich zum Strom f einzuspeisen. Dieser Strom kann aus allen butenhaltigen Quellen stammen. Denkbar sind z. B. FCC-Produktströme und durch Dimerisierung von Ethylen erzeugte butenhaltige Stoffströme.

Die Durchführung einzelner Stufen kann wie in DE-A-10 2004 059 356, DE-A-10 2004 054 766, bzw. DE-A-10 2004 061 514 beschrieben, erfolgen.

Nachstehend werden bevorzugte Verfahrensweisen beschrieben:

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₅⁺-Kohlenwasserstoffen. Die Zusammensetzung von LPG kann stark schwanken. Vorteilhafter Weise enthält das eingesetzte LPG mindestens 10 Gew.-% n-Butan.

Alternativ kann ein veredelter C₄-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
A1) Bereitstellung eines liquefied petroleum gas (LPG)-Stroms,
A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung der n-Butan/Isobutan-Gemische in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom kann einer Isomerisierung unterworfen werden. Dazu wird der Isobutan enthaltende Strom in einen Isomerisierungsreaktor eingespeist. Die Isomerisierung von Isobutan zu n-Butan kann wie in GB-A 2018815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether.

Der n-Butan enthaltende Einsatzgasstrom a enthält im Allgemeinen mindestens 60 Gew.-% n-Butan, vorzugsweise mindestens 90 Gew.-% n-Butan. Daneben kann er als Nebenbestandteile noch C₁-C₄-Kohlenwasserstoffe enthalten.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Butenen dehydriert, wobei auch Butadien (1,3-Butadien) gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Inertgase, wie Stickstoff im Produktgasgemisch der nicht-oxidativen katalytischen n-Butan-Dehydrierung enthalten sein. Daneben liegt im Produktgasgemisch nicht umgesetztes n-Butan vor.

Ein Merkmal der nicht-oxidativen Fahrweise gegenüber einer oxidativen Fahrweise ist, dass bei der oxidativen Dehydrierung kein freier Wasserstoff gebildet wird.

Die nicht-oxidative katalytische n-Butan-Dehydrierung kann grundsätzlich in allen aus dem Stand der Technik bekannten Reaktortypen und Fahrweisen durchgeführt werden. Eine Beschreibung von erfindungsgemäß geeigneten Dehydrierverfahren enthält auch "Catalytica® Studies Division, Oxidative Dehydrogenation and Alternative Dehydrogenation Processes" (Study Number 4192 OD, 1993,430 Ferguson Drive, Mountain View, California, 94043-5272, USA).

Die nicht-oxidative katalytische Butan-Dehydrierung kann mit oder ohne sauerstoffhaltigem Gas als Co-Feed durchgeführt werden. Vorzugsweise wird sie als autotherme nicht-oxidative Dehydrierung unter Einspeisung von Sauerstoff als Co-Feed durchgeführt. Bei der autothermen Fahrweise wird die benötigte Wärme direkt im Reaktorsystem durch Verbrennung von Wasserstoff und/oder Kohlenwasserstoffen in Gegenwart von Sauerstoff erzeugt. Vorzugsweise kann zusätzlich ein Wasserstoff enthaltender Co-Feed zugemischt werden. Dem Reaktionsgasgemisch der n-Butan-Dehydrierung wird in mindestens einer Reaktionszone zusätzlich Sauerstoff zugemischt und der in dem Reaktionsgasgemisch enthaltene Wasserstoff und/oder Kohlenwasserstoff wird zumindest teilweise verbrannt, wodurch zumindest ein Teil der benötigten Dehydrierwärme in der mindestens einen Reaktionszone direkt in dem Reaktionsgasgemisch erzeugt wird. Bevorzugt ist die Fahrweise mit reinem Sauerstoff. Sauerstoff kann bevorzugt als Sauerstoff-Dampf-Gemisch oder als Luft-Dampf-Gemisch eingespeist werden. Durch die Verwendung eines Sauerstoff-Dampfgemisches werden nur geringe Mengen an Inertgasen (Stickstoff) in den Gesamtprozess eingeschleust.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol.

Der zur Wärmeerzeugung verbrannte Wasserstoff ist der bei der katalytischen Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte so viel Wasserstoff zugegen sein, dass das Molverhältnis im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein spezieller Oxidationskatalysator erforderlich ist. Geeignete Katalysatoren sind zum Beispiel in DE-A 10 2004 061 514 beschrieben.

Als Reaktor sind alle dem Fachmann bekannten Reaktoren zum Einsatz heterogener Katalysatoren für Gas-Feststoff-Katalyse geeignet.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Geeignete Katalysatoren sind zum Beispiel in DE-A 10 2004 061 514 beschrieben, siehe auch WO 2009/124974 und WO 2009/124945 sowie besonders bevorzugt WO 2010/133565. Die Dehydriertemperatur beträgt im allgemeinen 400 bis 1100 °C, der Druck im Ausgang des Reaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Auch andere Reaktoren, wie Monolithreaktoren, sind geeignet.

Bevorzugt ist ein Reaktor (1) in Form eines liegenden Zylinders oder Prismas zur Durchführung einer autothermen Gasphasendehydrierung eines butanhaltigen Gasstromes (2) mit einem Sauerstoff enthaltenden Gasstrom (3) unter Erhalt eines Reaktionsgasgemisches, an einem heterogenen Katalysator, der als Monolith (4) ausgebildet ist, wobei
- der Innenraum des Reaktors (1) durch ein in Längsrichtung des Reaktors (1) angeordnetes kreiszylindrisches oder prismatisches gasdichtes Gehäuse G in
- einen Innenbereich A, mit einer oder mehreren katalytisch aktiven Zonen (5), worin jeweils eine Packung aus aufeinander, nebeneinander und hintereinander gestapelten Monolithen (4) und vor jeder katalytisch aktiven Zone (5) jeweils eine Mischzone (6) mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher (12) vorgesehen ist
- mit einer oder mehreren Zuführleitungen (7) für den zu dehydrieren-den butanhaltigen Gasstrom (2),
- mit einer oder mehreren, unabhängig voneinander regelbaren Zuführleitungen (9), wobei jede Zuführleitung (9) eine oder mehrere Verteilerkammern (10) versorgt, für den Sauerstoff enthaltenden Gasstrom (3) in jede der Mischzonen (6) sowie
- mit einer Abführleitung (11) für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagt ist und
- der zu dehydrierende butanhaltige Gasstrom (2) über ei-ne Zuführleitung (7) in den Wärmetauscher (12) eingeleitet, im Wärmetauscher (12) durch das Reaktionsgasgemisch im Gegenstrom durch indirekten Wärmetausch aufgeheizt und weiter an das dem Wärmetauscher (12) entgegengesetzte Ende des Reaktors ge-leitet, dort umgelenkt, über einen Strömungsgleichrichter (8) in den Innenbereich A eingeleitet, und in den Mischzonen (6) mit dem Sauerstoff enthaltenden Gasstrom (3) vermischt wird, worauf im Innenbereich A des Reaktors (1) die autotherme Gasphasendehydrierung stattfindet.

Bevorzugt ist dabei das unter den Reaktionsbedingungen der autothermen Gasphasendehydrierung inerte Gas Wasserdampf.

Bevorzugt wird dabei das unter den Reaktionsbedingungen der autothermen Gasphasendehydrierung inerte Gas als Purge-Gasstrom mit einem Massenstrom von 1/5 bis 1/100, bevorzugt 1/10 bis 1/50, bezogen auf den Massenstrom des butanhaltigen Gasstromes (2) unter geringem Überdruck von 2 bis 50 mbar, bevorzugt von 25 bis 30 mbar, bezogen auf den Druck im Innenbereich A durch den Außenbereich B geleitet, bevorzugt, indem der Purge-Gasstrom an einem Reaktorende über eine oder mehrere Zuführleitungen (20) in den Außenbereich B des Reaktors eingeleitet und am entgegengesetzten Reaktorende in den Innenbereich A des Reaktors weitergeleitet wird, insbesondere über eine oder mehrere, vorteilhaft in einem Winkel verschieden von 90° zur Zuführleitung (7) für den zu dehydrierenden butanhaltigen Gasstrom (2) angeordnete Verbindungsleitung(en) (21).

Bevorzugt wird dabei der zu dehydrierende butanhaltige Gasstrom (2), an zwei oder mehreren Stellen in den Wärmetauscher (12) eingeleitet, bevorzugt als ein Hauptstrom mit höherem Massenstrom und ein oder mehrere Nebenströme mit niedrigerem Massenstrom gegenüber dem Hauptstrom.

Bevorzugt sind dabei zusätzlich zum Wärmetauscher (12) eine oder mehrere Zusatzheizungen für den zu dehydrierenden butanhaltigen Gasstrom (2) vorgesehen.

Bevorzugt ist dabei als Zusatzheizung für den butanhaltigen Gasstrom (2) die Zuführung von Wasserstoff über eine Leitung (23) in die Zuführleitung (7) für den zu de-hydrierenden butanhaltigen Gasstrom (2), möglichst nahe am Eintritt in die Mischzonen (6), die vor jeder katalytisch aktiven Zone (5) an-geordnet sind, vorgesehen, wobei als Zusatzheizung eine elektrische Heizung (22) vorgesehen sein kann, die bevorzugt lösbar, als Einstecksystem, innerhalb des Außenbereichs B des Reaktors (1) eingebracht wird, oder als Muffelbrenner (22), in die Zuführleitung (7) für den zu dehydrierenden butanhaltigen Gasstrom (2) nach dem Austritt desselben aus dem Wärmetauscher (12).

Bevorzugt sind dabei im Innenbereich A zwei oder mehrere katalytisch aktive Zonen (5) mit jeweils einer Packung aus aufeinander, nebeneinander und hintereinander gestapelten Monolithen (4) vorgesehen.

Zwei oder mehrere der Reaktoren (1) können eingesetzt wer-den, wobei mindestens ein Reaktor (1) für die autotherme Gasphasendehydrierung genutzt und gleichzeitig mindestens ein weiterer Reaktor (1) regeneriert wird.

Die Regenerierung wird bevorzugt in einem Temperaturbereich zwischen 550 und 700°C durchgeführt.

Die Regenerierung wird bevorzugt mit einem sauerstoffhaltigen Gasstrom, enthaltend 0,1 bis 1,5 Gew.-% Sauerstoff, bezogen auf das Gesamtgewicht des sauerstoffhaltigen Gasstromes, durchgeführt.

In diesem Reaktor ist
- der Innenraum des Reaktors durch ein lösbar, in Längsrichtung des Reaktors angeordnetes kreiszylindrisches oder prismatisches, gasdichtes Gehäuse G in
- einen Innenbereich A, mit einer oder mehreren katalytisch aktiven Zonen, worin jeweils eine Packung aus aufeinander, nebeneinander und hintereinander gesta-pelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt, wobei
- an einem Reaktorende im Anschluss an das Gehäuse G ein Wärmetauscher vorgesehen ist
- mit einer oder mehreren Zuführleitungen für den zu dehydrierenden butanhaltigen Gasstrom,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgasgemisch der autothermen Gasphasendehydrierung,
wobei der Außenbereich B mit einem unter den Reaktionsbedingungen der autothermen Gasphasendehydrierung inerten Gas beaufschlagt ist und der zu dehydrierende butanstoffhaltige Gasstrom über eine Zuführleitung in den Wärmetauscher eingeleitet, durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet.

Die autotherme Gasphasendehydrierung findet dabei an einem heterogenen Katalysator statt, der in Form von Monolithen vorliegt.

Erfindungsgemäß werden die einzelnen Monolithe nebeneinander, übereinander und hintereinander, in der erforderlichen Anzahl, um eine katalytisch aktive Zone auszufüllen, unter Ausbildung einer Packung, gestapelt.

Vor jeder Packung ist jeweils eine Mischzone mit festen Einbauten, die nicht katalytisch aktiv sind, vorgesehen. In der Mischzone erfolgt die Vermischung des butanhaltigen Gasstromes mit dem Sauerstoff enthaltenden Strom, wobei in der in Strömungsrichtung zuerst angeströmten Mischzone die Vermischung des Sauerstoff enthaltenden Gasstromes mit dem butanhaltigen Einsatzstrom erfolgt und in den darauffolgend angeströmten Mischzonen jeweils eine Zwischenspeisung eines Sauerstoff enthaltenden Gasstromes in das noch zu dehydrierende, Butan enthaltende Reaktionsgemisch erfolgt.

Der zu dehydrierende butanhaltige Gasstrom kann bevorzugt an zwei oder mehreren Stellen in den Wärmetauscher eingeleitet werden, insbesondere als ein Hauptstrom mit höherem Massenstrom und ein oder mehrere Nebenströme mit niedrigerem Massenstrom gegenüber dem Hauptstrom.

Die Butan-Dehydrierung wird allgemein bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Bei der nicht-oxidativen katalytischen n-Butan-Dehydrierung wird ein Gasgemisch erhalten, das neben Butadien 1-Buten, 2-Butene und nicht umgesetztem n-Butan in der Regel Nebenbestandteile enthält. Übliche Nebenbestandteile sind Wasserstoff, Wasserdampf, CO₂, sowie Leichtsieder (Methan, Ethan, Ethen, Propan und Propen). Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält vorzugsweise 0,1 bis 15 Vol.-% Butadien, 1 bis 15 Vol.-% 1-Buten, 1 bis 25 Vol.% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.% Wasserstoff, 0 bis 10 Vol.-% Inertgas (Stickstoff) und 0 bis 5 Vol.-% Kohlenstoffoxide, wobei die Gesamtmenge der Inhaltsstoffe 100 Vol-% beträgt.

Der die erste Dehydrierzone verlassende Produktgasstrom b kann nach Kompression in Verfahrensstufe C) in Verfahrensstufe D) in zwei Teilströme aufgetrennt werden, wobei nur einer der beiden Teilströme den weiteren Verfahrensteilen E) bis M) unterworfen wird und der zweite Teilstrom in die erste Dehydrierzone zurückgeführt wird. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen E) bis M) unterworfen werden.

In der Verfahrensstufe C) wird der Gasstrom b vorzugsweise zunächst abgekühlt. Die Abkühlung des verdichteten Gases erfolgt mit Wärmetauschern, die beispielsweise als Rohrbündel-, Spiral- oder Plattenwärmetauscher ausgeführt sein können. Die dabei abgeführte Wärme wird bevorzugt zur Wärmeintegration im Verfahren genutzt. Anschließend wird in einer bevorzugten Ausführungsform des Verfahrensschrittes C) aus dem Produktstrom Wasser abgetrennt. Die Abtrennung des Wassers erfolgt dabei vorzugsweise in einem Quench.

Anschließend wird der Gasstrom c in mindestens einer ersten Kompressionsstufe komprimiert und nachfolgend abgekühlt, wobei mindestens ein Kondensatstrom c1 enthaltend Wasser auskondensiert und ein Gasstrom c2 enthaltend n-Butan, 1-Buten, 2-Butene, Butadien, Wasserstoff, Wasserdampf, in geringen Mengen Methan, Ethan, Ethen, Propan und Propen gegebenenfalls Kohlenstoffoxide und gegebenenfalls Inertgase verbleibt.

Die Kompression kann ein- oder mehrstufig erfolgen. Insgesamt wird von einem Druck im Bereich von 1,0 bis 4,0 bar auf einen Druck im Bereich von 3,5 bis 20 bar komprimiert. Nach jeder Kompressionsstufe folgt eine Abkühlstufe, in der der Gasstrom auf eine Temperatur im Bereich von 15 bis 60°C abgekühlt wird. Der Kondensatstrom c1 kann somit bei mehrstufiger Kompression auch mehrere Ströme umfassen.

Der Gasstrom c2 besteht im Allgemeinen im Wesentlichen aus C₄-Kohlenwasserstoffen (im Wesentlichen n-Butan, 1-Buten und 2-Butene), Wasserstoff, Kohlendioxid und Wasserdampf. Daneben kann der Strom c2 noch Leichtsieder, Butadien und Inertgase (Stickstoff) als weitere Nebenkomponenten enthalten. Der Kondensatstrom c1 besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlenstoffoxide.

Geeignete Verdichter sind beispielsweise Turbo-, Drehkolben- und Hubkolbenverdichter. Die Verdichter können beispielsweise mit einem Elektromotor, einem Expander oder einer Gas-oder Dampfturbine angetrieben werden. Typische Verdichtungsverhältnisse (Austrittsdruck: Eintrittsdruck) pro Verdichterstufe liegen je nach Bauart zwischen 1,5 und 3,0.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei in der Regel Kühlwasser oder Wärmeträgeröle zum Einsatz. Daneben wird bevorzugt Luftkühlung unter Einsatz von Gebläsen eingesetzt.

Die Absorption in Schritt (D) kann in jeder beliebigen, dem Fachmann bekannten geeigneten Absorptionskolonne durchgeführt werden. Bevorzugt wird die Absorption im Gegenstrom durchgeführt. Hierzu wird der Absorptionskolonne im unteren Bereich der Butene, Butadien, Butan, Wasserstoff, Inertgas (Stickstoff) und gegebenenfalls Kohlenstoffoxide enthaltende Stoffstrom zugeführt. Im oberen Bereich der Adsorptionskolonne wird der N-Methylpyrrolidon und Wasser enthaltende Stoffstrom aufgegeben.

Am Kopf der Absorptionskolonne wird ein wasserstoffreicher und/oder inertgas(stickstoff)reicher Stoffstrom d2 entnommen, der gegebenenfalls noch Reste an C₄-Kohlenwasserstoffen und gegebenenfalls Kohlenstoffoxigenate enthält. Weiterhin kann dieser Strom Inerte (beispielsweise Stickstoff) und Leichtsieder (Ethan, Ethen, Propan, Propen, Methan) enthalten. Der N-Methylpyrrolidon und Wasser enthaltende Stoffstrom kühlt den zugeführten Butene und/oder Butadien, Butan, Wasserstoff und/oder Inertgas (Stickstoff) und gegebenenfalls Kohlenstoffoxide enthaltenden Stoffstrom ab und absorbiert gleichzeitig bevorzugt die C₄-Komponenten und teilweise Kohlenstoffoxide. Gegebenenfalls werden auch kleine Mengen an H₂, Inerten (N₂) und Leichtsiedern absorbiert. Dieser Strom wird am Sumpf der Absorptionskolonne abgezogen.

Die Verwendung einer Mischung von N-Methylpyrrolidon und Wasser als Lösungsmittel für die Absorption und als Extraktionsmittel in der Extraktivdestillation hat den Vorteil, dass die Siedetemperatur niedriger liegt als die Siedetemperatur bei Verwendung von reinem N-Methylpyrrolidon. Ein weiterer Vorteil ist, dass durch Zunahme des Wasseranteils in der als Lösungsmittel eingesetzten Mischung aus Wasser und N-Methylpyrrolidon die Selektivität gesteigert werden kann. Dies führt jedoch erwartungsgemäß zu einer Verringerung der Kapazität. Ein weiterer Vorteil ist die Selektivität des N-Methylpyrrolidons gegenüber Kohlenstoffoxiden, insbesondere Kohlendioxid. Dies ermöglicht zusätzlich zur Abtrennung der Kohlenwasserstoffe eine Abtrennung der Kohlenstoffoxide, insbesondere des Kohlendioxids vom Wasserstoff.

Die Absorption in Schritt D) wird im Allgemeinen bei einer Sumpftemperatur im Bereich von 30 bis 160 °C, einer Kopftemperatur im Bereich von 5 bis 60°C und bei einem Druck im Bereich von 2 bis 20 bar durchgeführt. Bevorzugt wird die Absorption bei einer Sumpftemperatur im Bereich von 30 bis 100°C, bei einer Kopftemperatur im Bereich von 25 bis 50 °C und einem Druck im Bereich von 8 bis 15 bar durchgeführt.

Die Absorptionskolonne ist vorzugsweise eine Füllkörperkolonne oder eine Packungskolonne. Es ist aber auch jede beliebige andere Kolonne, zum Beispiel eine Bodenkolonne denkbar. Eine für die Absorption geeignete Kolonne weist vorzugsweise 2 bis 40 theoretische Trennstufen, bevorzugt 5 bis 25 theoretische Trennstufen auf.

Die Temperatur des N-Methylpyrrolidon und Wasser enthaltenden Stoffstroms, z. B. e1 und/oder k2 der der Absorptionskolonne zugeführt wird, liegt vorzugsweise bei 10 bis 70°C, bevorzugt bei 20 bis 40°C. Die Temperatur des Butene, Butadien, Butan, Wasserstoff und/oder Inertgas (Stickstoff) und gegebenenfalls Kohlenstoffoxide enthaltenden Stoffstroms liegt vorzugsweise im Bereich zwischen 0 und 400 °C, insbesondere im Bereich zwischen 40 und 200 °C.

Das Verhältnis von eingesetztem N-Methylpyrrolidon zum Butene, Butadien, Butan, Wasserstoff und/oder Inertgas und gegebenenfalls Kohlenstoffoxide enthaltendem Stoffstrom liegt vorzugsweise im Bereich von 2 bis 30, mehr bevorzugt im Bereich von 4 bis 30 und insbesondere im Bereich von 4 bis 15, jeweils bezogen auf die Massen der eingesetzten Stoffströme.

Der bei der Absorption anfallende N-Methylpyrrolidon, Wasser, Butene, Butadien, Butan und Kohlenstoffoxide enthaltende Stoffstrom d1 enthält im Allgemeinen 20 bis 90 mol-% N-Methylpyrrolidon, 0 bis 50 mol-% Wasser, 0 bis 20 mol-% Butadien, 0 bis 20 mol-% 1-Buten, 0 bis 20 mol-% 2-Butene, 0 bis 50 mol-% Butan und 0 bis 20 mol-% Kohlenstoffoxide.

Der bei der Absorption erhaltene N-Methylpyrrolidon, Wasser, Butene, Butadien, Butan und Kohlenstoffoxide enthaltende Stoffstrom d1 wird dann in Schritt (E) einer Extraktivdestillation zugeführt.

Die Extraktivdestillation kann beispielsweise wie in Erdöl und Kohle Erdgas -Petrochemie Band 34 (8). Seiten 343 bis 346 oder Ullmanns Enzyklopädie der technischen Chemie. Band 9. 4. Auflage 1975. Seiten 1 bis 18 beschrieben durchgeführt werden.

In der Extraktivdestillation wird der Butene, Butadien, Butan, Methylpyrrolidon, Wasser und Kohlenstoffoxide enthaltende Stoffstrom d1 mit einem N-Methylpyrrolidon und Wasser enthaltenden Stoffstrom in einer Extraktivdestillationszone in Kontakt gebracht. Die Extraktivdestillationszone ist im Allgemeinen in Form einer Kolonne ausgeführt, die Böden, Füllkörper oder Packungen als Einbauten enthält. Die Extraktivdestillationszone weist im Allgemeinen 10 bis 70 theoretische Böden auf, damit eine hinreichend gute Trennwirkung erzielt wird. Vorzugsweise weist die Extraktionskolonne im Kolonnenkopf eine Rückwaschzone auf. Diese Rückwaschzone dient zur Rückgewinnung des in der Gasphase enthaltenen N-Methylpyrrolidons durch flüssigen Kohlenwasserstoffrücklauf, wozu die Kopffraktion zuvor kondensiert wird. Typische Temperaturen am Kopf der Kolonne liegen zwischen 30 und 60 °C.

Der Kopfproduktstrom e3 der Extraktivdestillationskolonne enthält Butan und Kohlenstoffoxide und wird gasförmig abgezogen. Neben Butan und Kohlenstoffoxide können weiterhin Butene, Wasserstoff und/oder Inertgas und andere Leichtsieder im Kopfproduktstrom enthalten sein. In einer bevorzugten Ausführungsform wird der Kopfproduktstrom e3 kondensiert, um Kohlenstoffoxide wie CO₂, sowie gegebenenfalls enthaltenen Wasserstoff und/oder Inertgas und Leichtsieder von Butan abzutrennen. Der flüssige Butanstrom kann zum Beispiel in die Dehydrierzone im Verfahrensschritt B) zurückgeführt werden.

Am Sumpf der Extraktivdestillationskolonne wird ein N-Methylpyrrolidon, Wasser, Butene, Butan und Butadien enthaltender Stoffstrom e2 gewonnen. Durch Abtrennung eines Teils des Butans über Kopf erfolgt hier eine Aufkonzentrierung der Butene im Stoffstrom e2. Der Grad der Aufkonzentrierung ist durch die Parametrisierung der Kolonne einstellbar.

Der N-Methylpyrrolidon, Wasser, Butan, Butene und Butadien enthaltende Strom e2, der am Sumpf der Extraktivdestillationskolonne gewonnen wird, wird einer Destillationskolonne F) zugeführt, in der über Kopf ein Stoffstrom f bestehend aus im wesentlichen Butenen, Butan und Butadien gewonnen werden. Am Sumpf der Destillationskolonne fällt ein N-Methylpyrrolidon und Wasser enthaltender Stoffstrom e1) an, wobei die Zusammensetzung des N-Methylpyrrolidon und Wasser enthaltenden Stoffstroms der Zusammensetzung entspricht, wie sie der Absorption und der Extraktion zugegeben wird. Der N-Methylpyrrolidon und Wasser enthaltende Stoffstrom wird vorzugsweise aufgeteilt und zurück in die Absorption im Verfahrensschritt D) und die Extraktivdestillation im Verfahrensschritt E) geleitet. Das Verhältnis des Gemischs aus Wasser und N-Methylpyrrolidon, das der Absorption zugeführt wird, zu dem Gemisch aus Wasser und N-Methylpyrrolidon und C₄, das der Extraktivdestillation zugeführt wird, liegt vorzugsweise im Bereich von 0,2 bis 20, insbesondere im Bereich von 0,3 bis 15.

Der über Kopf abgetrennte Stoffstrom f kann teilweise oder vollständig aus der Anlage abgezogen und als Produktstrom verwendet werden. Dabei kann der Butengehalt über die Betriebsweise der Extraktivdestillation eingestellt werden. Eine hohe Butenkonzentration verringert die Butanmenge die durch Verfahrensschritt G) und die nachfolgenden Verfahrensschritte geführt werden muss. Gleichzeitig wird hierdurch die Ausbeute der BDH-Stufe erhöht.

Die Extraktivdestillation wird vorzugsweise bei einer Sumpftemperatur im Bereich von 90 bis 250°C, insbesondere bei einer Temperatur im Bereich von 90 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Destillation im Verfahrensschritt (F) wird vorzugsweise bei einer Sumpftemperatur im Bereich von 100 bis 300 °C, insbesondere im Bereich von 150 bis 200°C und einer Kopftemperatur im Bereich von 0 bis 70° C, insbesondere im Bereich von 10 bis 50° C durchgeführt. Der Druck in der Destillationskolonne liegt dabei vorzugsweise im Bereich von 1 bis 10 bar. Die Destillationskolonne weist vorzugsweise 2 bis 30, insbesondere 5 bis 20 theoretische Trennstufen auf.

Neben dem aus Verfahrensschritt F) erhaltenen Stoffstrom f können auch weitere n-butenhaltige Stoffströme der ODH-Stufe in Verfahrensschritt G) zugeführt werden, wie sie beispielsweise in Raffinerien aus FCC-Einheiten oder durch Dimerisierung von Ethylen erhalten werden. Erfindungsgemäß ist die Zusetzung jedes n-butenhaltigen Stoffstromes denkbar.

In der oxidativen (katalytischen) Dehydrierung im Verfahrensschritt G) werden im Wesentlichen 1-Buten und 2-Butene zu 1,3-Butadien dehydriert, wobei 1-Buten im Allgemeinen nahezu vollständig abreagiert.

Die oxidative Dehydrierung kann grundsätzlich in allen aus dem Stand der Technik bekannten Reaktortypen und nach bekannten Fahrweisen durchgeführt werden, beispielsweise im Wirbelbett, im Hordenofen, im Festbettrohr- oder Rohrbündelreaktor oder im Plattenwärmetauscherreaktor. Zur Durchführung der oxidativen Dehydrierung wird vorzugsweise ein Gasgemisch benötigt, das ein molares Sauerstoff: n-Butene-Verhältnis von mindestens 0,5 aufweist. Bevorzugt wird bei einem Sauerstoff: n-Butene-Verhältnis von 0,55 bis 50, vorzugsweise 0,55 bis 10, insbesondere 0,55 bis 3 gearbeitet. Zur Einstellung dieses Wertes wird in der Regel das aus der nicht-oxidativen katalytischen Dehydrierung stammende Produktgasgemisch direkt oder nach einer Aufarbeitung, bei der Butene aufkonzentriert und Wasserstoff abgetrennt werden, mit reinem Sauerstoff oder einem sauerstoffhaltigen Gas vermischt. In einer Ausführungsform des Verfahrens ist das sauerstoffhaltige Gas Luft. Das erhaltene sauerstoffhaltige Gasgemisch wird dann der Oxidehydrierung zugeführt. Alternativ und bevorzugt gegenüber Luft, ist es, zusätzlichen Stickstoff oder Magerluft mit einem Anteil von weniger als 23 Vol.-% als sauerstoffhaltiges Gas einzusetzen. In einer bevorzugten Ausführungsform wird das Abgas aus Verfahrensschritt J) Stoffstrom j2 mit dem Stoffstrom f und gegebenenfalls zusätzlichem Dampf vermischt und dem Verfahrensschritt G) zugeführt. Damit kann die zur Verdünnung des Stoffstroms f gegebenenfalls benötigte Stickstoffmenge reduziert oder überflüssig gemacht werden.

Die für die Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281, US 4,336,409, DE-A-2600128 und DE-A-2440329 sowie WO 2009/124974 und WO 2009/124945.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als so genannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Des Weiteren ist es möglich, die Aktivmassen der Katalysatoren auf einen Träger, beispielsweise einen anorganischen, oxidischen Formkörper, inklusive eventueller Hilfsmittel, wie etwa Graphit oder Porenbildner, sowie weiterer Komponenten aufzubringen. Derartige Katalysatoren werden in der Regel als Schalenkatalysatoren bezeichnet.

Die Oxidehydrierung wird im Allgemeinen bei einer Temperatur von 220 bis 490 °C und bevorzugt von 250 bis 450 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Der die oxidative Dehydrierung verlassende Produktgasstrom g enthält neben Butadien und nicht in Verfahrensschritt E) abgetrenntem n-Butan noch Wasserstoff, Kohlenstoffoxide und Wasserdampf. Als Nebenbestandteile kann er noch Sauerstoff, Inertgas, wie Stickstoff, Methan, Ethan, Ethen, Propan und Propen sowie sauerstoffhaltige Kohlenwasserstoffe, sogenannte Oxigenate, enthalten.

Im Allgemeinen weist der die oxidative Dehydrierung verlassende Produktgasstrom g 2 bis 40 Vol.-% Butadien, 5 bis 80 Vol.-% n-Butan, 0 bis 15 Vol.-% 2-Butene, 0 bis 5 Vol.-1-Buten, 5 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 70 Vol.-% Inertgas, 0 bis 10 Vol.-% Kohlenstoffoxide, 0 bis 10 Vol.-% Sauerstoff und 0 bis 10 Vol.-% Oxygenate auf, wobei die Gesamtmenge der Inhaltsstoffe 100 Vol-% ergibt. Oxygenate können beispielsweise Furan, Essigsäure, Methacrolein, Maleinsäureanhydrid, Maleinsäure, Phthalsäureanhydrid, Propionsäure, Acetaldehyd, Acrolein, Formaldehyd, Ameisensäure, Benzaldehyd, Benzoesäure und Butyraldehyd sein. Daneben können in Spuren Acetylen, Propin und 1,2-Butadien enthalten sein.

Enthält der Produktgasstrom g mehr als nur geringfügige Spuren Sauerstoff, so wird im Allgemeinen eine Verfahrensstufe H) zur Entfernung von Rest-Sauerstoff aus dem Produktgasstrom g durchgeführt. Der Rest-Sauerstoff kann sich insoweit als störend auswirken, als er in nachgelagerten Verfahrensschritten eine Butadienperoxidbildung hervorrufen kann und als Initiator für Polymerisationsreaktionen wirken kann.

Unstabilisiertes 1,3-Butadien kann in Gegenwart von Sauerstoff gefährliche Butadienperoxide bilden. Die Peroxide sind viskose Flüssigkeiten. Ihre Dichte ist höher als die von Butadien. Da sie außerdem nur wenig in flüssigem 1,3-Butadien löslich sind, setzen sie sich auf den Böden von Lagerbehältern ab. Trotz ihrer relativ geringen chemischen Reaktivität sind die Peroxide sehr instabile Verbindungen, die sich bei Temperaturen zwischen 85 und 110 °C spontan zersetzen können. Eine besondere Gefahr besteht in der hohen Schlagempfindlichkeit der Peroxide, die mit der Brisanz eines Sprengstoffes explodieren.

Die Gefahr der Polymerbildung ist insbesondere bei der destillativen Abtrennung von Butadien (Schritte L und M) gegeben und kann dort zu Ablagerungen von Polymeren (Bildung von so genanntem "Popcorn") in den Kolonnen führen.

Vorzugsweise wird die Sauerstoffentfernung H) unmittelbar nach der oxidativen Dehydrierung G) durchgeführt. Im Allgemeinen wird hierzu eine katalytische Verbrennungsstufe durchgeführt, in der Sauerstoff mit in dieser Stufe zugesetztem Wasserstoff in Gegenwart eines Katalysators umgesetzt wird. Dieser Wasserstoff kann als Teil des Stoffstrom d2 aus Verfahrensstufe D) entnommen werden. Hierdurch wird eine Verringerung des Sauerstoffgehalts bis auf geringe Spuren erreicht.

Der freien Sauerstoff enthaltende Kohlenwasserstoffstrom kann eine zur Umsetzung mit dem freien Sauerstoff ausreichende Menge an freiem Wasserstoff enthalten. Fehlende Mengen oder die Gesamtmenge an benötigtem freiem Wasserstoff kann/können auf dem Kohlenwasserstoffstrom zugesetzt werden. Bei dieser Reaktionsführung kann der freie Sauerstoff mit dem freien Wasserstoff umgesetzt werden, so dass kein nennenswerter Anteil des Kohlenwasserstoffs mit dem Sauerstoff umgesetzt wird.

In einer alternativen Ausführungsform enthält der freien Sauerstoff enthaltende Kohlenwasserstoffstrom keinen freien Wasserstoff und ihm wird auch kein freier Wasserstoff zugesetzt. In diesem Fall kann der freie Sauerstoff mit dem im freien Sauerstoff enthaltenden Kohlenwasserstoffstrom enthaltenden Kohlenwasserstoff oder mit zugesetztem Methanol, Erdgas und/oder Synthesegas als Reduktionsmittel umgesetzt werden.

Die Verfahrensführung kann dabei isotherm oder adiabatisch sein. Vorteil der Umsetzung des Wasserstoffs ist die Bildung von Wasser als Reaktionsprodukt. Das gebildete Wasser lässt sich durch Auskondensieren leicht abtrennen.

Da Butadien ein reaktives Molekül ist, sind niedrige Umsetzungstemperaturen bei der Sauerstoffentfernung von Vorteil. Hierdurch können hohe Selektivitäten erreicht werden und unkontrollierte homogene Reaktionen können vermieden werden.

Zudem kann ein niedriger Reaktionsdruck von Vorteil sein, da hierdurch ein gesonderter Verdichtungsschritt nach der oxidativen Dehydrierung vermieden werden kann. Ein niedrigerer Umsetzungsdruck erlaubt eine weniger aufwendige Reaktorfertigung und ist aus Sicherheitsgründen vorteilhaft.

Das erfindungsgemäße Verfahren wird daher in Schritt H) vorzugsweise bei einem Druck von 0,5 bis 3,0 bar (absolut), besonders bevorzugt 1,0 bis 2,0 bar (absolut) durchgeführt.

Die Umsetzung erfolgt vorzugsweise bei einer Temperatur im Bereich von 100 bis 650 °C, besonders bevorzugt 250 bis 550 °C.

Der Reaktortyp ist erfindungsgemäß nicht eingeschränkt. Beispielsweise kann die Umsetzung im Wirbelbett, im Hordenofen, im Festrohr- oder Rohrbündelreaktor oder im Plattenwärmetauscherreaktor erfolgen. Auch eine Kaskadierung von Wirbelschicht-Reaktoren ist denkbar.

Die bei der Reaktion entstehende Wärme kann über die Reaktorwände abgeführt werden. Zudem kann durch Strukturierung eines Festbettes des Katalysators mit Inert-Materialien die Bildung von hot spots vermindert werden.

Wird im erfindungsgemäßen Verfahren in Schritt H) Wasserstoff im Überschuss eingesetzt, so kann die Reaktion mit Wasserstoff dazu dienen, eine ausreichend hohe Temperatur für die notwendige Reaktion zwischen Kohlenwasserstoffen und Sauerstoff zu erreichen. Hierdurch kann eine Verkokung weitgehend vermieden werden.

Wird kein Wasserstoff, bzw. ein Wasserstoff-Unterschuss eingesetzt, so reagiert der Sauerstoff vorwiegend mit dem reaktivsten Molekül, beispielsweise Butadien. Hierdurch kommt es zu einer Bildung von Kohlenstoffoxiden und Wasser. Da die Umsetzung von Sauerstoff mit den Kohlenwasserstoffen bei niedriger Temperatur langsamer als mit Wasserstoff abläuft, wird zunächst der Wasserstoff vollständig verbraucht.

Eine weitere erfindungsgemäße Ausführungsform ist die Durchführung dieser katalytischen Reaktion zusammen mit der oxidativen Dehydrierung in Verfahrensschritt G in einem Reaktor mit 2 Katalysatoren und gegebenenfalls Zwischeneinspeisung des Verbrennungsgases nach der Dehydrierschüttung.

In der Verfahrensstufe I) wird der Gasstrom h vorzugsweise zunächst abgekühlt. Die Abkühlung des verdichteten Gases erfolgt mit Wärmetauschern, die beispielsweise als Rohrbündel-, Spiral- oder Plattenwärmetauscher ausgeführt sein können. Die dabei abgeführte Wärme wird bevorzugt zur Wärmeintegration im Verfahren genutzt. Anschließend wird in einer bevorzugten Ausführungsform des Verfahrensschrittes I) aus dem Produktstrom Wasser abgetrennt. Die Abtrennung des Wassers erfolgt dabei vorzugsweise in einem Quench. Der Quench dient zudem der Abtrennung von sauerstoffhaltigen Nebenprodukten.

Anschließend wird der Gasstrom h in mindestens einer ersten Kompressionsstufe komprimiert und nachfolgend abgekühlt, wobei mindestens ein Kondensatstrom i1 enthaltend Wasser auskondensiert und ein Gasstrom i2 enthaltend n-Butan, 1-Buten, 2-Butene, Butadien, gegebenenfalls Wasserstoff, Wasserdampf, in geringen Mengen Methan, Ethan, Ethen, Propan und Propen, Kohlenstoffoxide und Inertgase verbleibt.

Die Kompression kann ein- oder mehrstufig erfolgen. Insgesamt wird von einem Druck im Bereich von 1,0 bis 4,0 bar auf einen Druck im Bereich von 3,5 bis 20 bar komprimiert. Nach jeder Kompressionsstufe folgt eine Abkühlstufe, in der der Gasstrom auf eine Temperatur im Bereich von 15 bis 60°C abgekühlt wird. Der Kondensatstrom i1 kann somit bei mehrstufiger Kompression auch mehrere Ströme umfassen.

Der Kondensatstrom i1 besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlenstoffoxide.

Geeignete Verdichter sind beispielsweise Turbo-, Drehkolben- und Hubkolbenverdichter. Die Verdichter können beispielsweise mit einem Elektromotor, einem Expander oder einer Gas-oder Dampfturbine angetrieben werden. Typische Verdichtungsverhältnisse (Austrittsdruck: Eintrittsdruck) pro Verdichterstufe liegen je nach Bauart zwischen 1,5 und 3,0.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, Butene, Butan, Wasserstoff, Inertgas und gegebenenfalls Kohlenstoffoxide sowie leicht siedende Kohlenwasserstoffe (Methan, Ethan, Ethen, Propan, Propen) enthaltende Stoffstrom i2, wird als Ausgangsstrom dem Schritt J) der Aufbereitung zugeführt.

In einer bevorzugten Ausführungsform des erfindungsgemäßen Verfahrens werden die nicht kondensierbaren oder leicht siedenden Gasbestandteile wie Wasserstoff, Sauerstoff, Kohlenstoffoxide, die leicht siedenden Kohlenwasserstoffe (Methan, Ethan, Ethen, Propan, Propen) und Inertgas, wie Stickstoff in einem Absorptions/Desorptions-Cyclus mittels eines hoch siedenden Absorptionsmittels abgetrennt, wobei ein C₄-Produktgasstrom j1 erhalten wird, der im Wesentlichen aus den C₄-Kohlenwasserstoffen besteht. Im Allgemeinen besteht der C₄-Produktgasstrom j1 zu mindestens 80 Vol.-%, bevorzugt zu mindestens 90 Vol.-%, besonders bevorzugt zu mindestens 95 Vol.-% aus den C₄-Kohlenwasserstoffen. Im Wesentlichen besteht der Strom j1 aus n-Butan, Butenen, wie 2-Butenen und Butadien.

Dazu wird in einer Absorptionsstufe der Produktgasstrom i2 nach vorheriger Wasserabtrennung mit einem inerten Absorptionsmittel in Kontakt gebracht und es werden die C₄-Kohlenwasserstoffe in dem inerten Absorptionsmittel absorbiert, wobei mit C₄-Kohlenwasserstoffen beladenes Absorptionsmittel und ein die obrigen Gasbestandteile enthaltendes Abgas j2 erhalten werden. In einer Desorptionsstufe werden die C₄-Kohlenwasserstoffe aus dem Absorptionsmittel wieder freigesetzt.

Die Absorptionsstufe in Schritt J) kann in jeder beliebigen, dem Fachmann bekannten geeigneten Absorptionskolonne durchgeführt werden. Die Absorption kann durch einfaches Durchleiten des Produktgasstroms i2 durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z.B. Blechpackungen mit einer spezifischen Oberflache von 100 bis 1000 m₂/m₃ wie Mellapak® 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

In einer erfindungsgemäßen Ausführungsform wird einer Absorptionskolonne im unteren Bereich der Butadien, Buten, Butan, Wasserstoff und/oder Stickstoff und gegebenenfalls Kohlendioxid enthaltende Stoffstrom zugeführt. Im oberen Bereich der Absorptionskolonne wird der Lösungsmittel und ggf. Wasser enthaltende Stoffstrom aufgegeben.

Erfindungsgemäße Absorptionsmittel sind Octane, Nonane, Decane, Undecane, Dodecane, Tridecane, Tetradecane, Pentadecane, Hexadecane, Heptadecane und Octadecane oder aus Raffineriestromen gewonnene Fraktionen, die als Hauptkomponenten die genannten linearen Alkane enthalten.

Geeignete Absorptionsmittel sind weiterhin vergleichsweise unpolare organische Losungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmetrageröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

In einer bevorzugten Ausführungsform wird als Lösungsmittel für die Absorption in Schritt J) ein Alkangemisch, wie Tetradekan (technischer C14-C17 Schnitt) eingesetzt.

Am Kopf der Absorptionskolonne wird ein Abgasstrom j2 abgezogen, der im wesentlichen Inertgas, Kohlenstoffoxide, gegebenenfalls Butan, Butene, wie 2-Butene und Butadien ggf. Wasserstoff und leicht siedende Kohlenwasserstoffe (Methan, Ethan, Ethen, Propan, Propen) und Wasserdampf enthält. Dieser Stoffstrom j2 wird dem Verfahrensschritt G) zugeführt. Damit lässt sich der Eintrittsstrom des ODH Reaktors auf den gewünschten C₄-Gehalt einstellen.

Der mit C₄-Kohlenwasserstoffen beladene Lösungsmittelstrom wird in eine Desorptionskolonne geleitet. Erfindungsgemäß sind alle dem Fachmann bekannten Kolonneneinbauten für diesen Zweck geeignet. In einer Verfahrensvariante wird der Desorptionsschritt durch Entspannung und/oder Erhitzen des beladenen Lösungsmittels durchgeführt. Bevorzugte Verfahrensvariante ist die Zugabe von Strippdampf und/oder die Zufuhr von Frischdampf im Sumpf des Desorbers. Das C₄-abgereicherte Lösungsmittel kann als Gemisch gemeinsam mit dem kondensierten Dampf (Wasser) einer Phasentrennung zugeführt werden, so dass das Wasser vom Lösungsmittel abgeschieden wird. Alle dem Fachmann bekannten Apparate sind hierfür geeignet. Möglich ist zudem die Nutzung des vom Lösungsmittel abgetrennten Wassers zur Erzeugung des Strippdampfes. Bevorzugt werden 70 bis 100 Gew.-% Lösungsmittel und 0 bis 30 Gew.-% Wasser, besonders bevorzugt 80 bis 100 Gew.-% Lösungsmittel und 0 bis 20 Gew.-% Wasser, insbesondere 85 bis 95 Gew.-% Lösungsmittel und 5 bis 15 Gew.-% Wasser eingesetzt.

Die Abtrennung J) ist im Allgemeinen nicht ganz vollständig, so dass in dem C₄-Produktgasstrom - je nach Art der Abtrennung - noch geringe Mengen oder auch nur Spuren der weiteren Gasbestandteile, insbesondere der leicht siedenden Kohlenwasserstoffe, vorliegen können. Die durch die Abtrennung J) auch bewirkte Volumenstromverringerung entlastet die nachfolgenden Verfahrensschritte.

Der im Wesentlichen aus n-Butan, Butenen, wie 2-Butenen und Butadien bestehende C₄-Produktgasstrom j1 enthält im Allgemeinen 20 bis 80 Vol.-% Butadien, 20 bis 80 Vol.-% n-Butan, 0 bis 10 Vol.-% 1-Buten, und 0 bis 50 Vol.-% 2-Butene, wobei die Gesamtmenge 100 Vol.-% ergibt.

In einem Verfahrensteil K) wird der C₄-Produktgasstrom j1 in einen im Wesentlichen aus n-Butan und Butenen, wie 2-Butenen bestehenden Rückführstrom k2 und einen im Wesentlichen aus Butadien bestehenden Strom k1 durch Extraktivdestillation getrennt. Der Strom k2 wird bevorzugt dem Einsatzgasstrom in Schritt A) zugegeben, in die Absorptionsstufe in Verfahrensschritt D), den Extraktionsschritt E) und/oder in Verfahrensschritt G) (ODH-Reaktor) (teilweise) zurückgeführt.

Die Extraktivdestillation K) kann beispielsweise wie in Erdöl und Kohle Erdgas -Petrochemie Band 34 (8). Seiten 343 bis 346 oder Ullmanns Enzyklopädie der technischen Chemie. Band 9. 4. Auflage 1975. Seiten 1 bis 18 beschrieben durchgeführt werden.

Die Extraktivdestillation wird vorzugsweise bei einer Sumpftemperatur im Bereich von 100 bis 250·C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

In der Extraktivdestillation wird der Butene, Butadien, Butan, Methylpyrrolidon, und Wasser enthaltende Stoffstrom mit einem wie vorstehend beschriebenen N-Methylpyrrolidon und Wasser enthaltenden Stoffstrom in einer Extraktivdestillationszone in Kontakt gebracht. Die Extraktivdestillationszone ist im Allgemeinen in Form einer oder mehrerer Kolonne(n) ausgeführt, die Böden, Füllkörper oder Packungen als Einbauten enthält. Die Extraktivdestillationszone weist im Allgemeinen 10 bis 70 theoretische Böden auf, damit eine hinreichend gute Trennwirkung erzielt wird. Vorzugsweise weist die Extraktionskolonne im Kolonnenkopf eine Rückwaschzone auf. Diese Rückwaschzone dient zur Rückgewinnung des in der Gasphase enthaltenen N-Methylpyrrolidons durch flüssigen Kohlenwasserstoffrücklauf, wozu die Kopffraktion zuvor kondensiert wird. Typische Temperaturen am Kopf der Kolonne liegen zwischen 30 und 60 °C.

Der Kopfproduktstrom k2 der Extraktivdestillationskolonne enthält im Wesentlichen Butan und Butene und in geringen Mengen Butadien und wird gasförmig oder flüssig abgezogen. In einer bevorzugten Ausführungsform wird der Kopfproduktstrom kondensiert, um Kohlenstoffoxide, wie CO₂ abzutrennen. Der flüssige Butan/Butenstrom kann in die Absorptionskolonne in Verfahrensschritt A), D), E) und/oder in Stufe G) zurückgeführt werden. Dadurch gelangt dieser Stoffstrom zusammen mit dem gequenchten, gekühlten, verdichteten und von Kondensat befreiten Produktgas der ersten Dehydrierstufe in die Extraktivdestillation zur Auftrennung on Butanen und Butenen.

Diese Auftrennung von Butan und Buten muss dann nicht neben der Butadienabtrennung in der zweiten Extraktivdestillation durchgeführt werden.

Am Sumpf der Extraktivdestillationskolonne wird ein N-Methylpyrrolidon, Wasser, Butadien und in geringen Anteilen Butene, Butan enthaltender Stoffstrom k1 gewonnen, der einer Destillationskolonne L) zugeführt wird. In dieser wird über Kopf oder als Seitenabzug Butadien gewonnen werden. Am Sumpf der Destillationskolonne fällt ein N-Methylpyrrolidon und Wasser enthaltender Stoffstrom I1 an, wobei die Zusammensetzung des N-Methylpyrrolidon und Wasser enthaltenden Stoffstroms der Zusammensetzung entspricht, wie sie der Extraktion zugegeben wird. Der N-Methylpyrrolidon und Wasser enthaltende Stoffstrom wird bevorzugt in die Extraktivdestillation im Verfahrensschritt K) geleitet.

Die Extraktivdestillation wird vorzugsweise bei einer Sumpftemperatur im Bereich von 90 bis 250°C, insbesondere bei einer Temperatur im Bereich von 90 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Destillation im Verfahrensschritt L) wird vorzugsweise bei einer Sumpftemperatur im Bereich von 100 bis 300 °C, insbesondere im Bereich von 150 bis 200°C und einer Kopftemperatur im Bereich von 0 bis 70° C, insbesondere im Bereich von 10 bis 50° C durchgeführt. Der Druck in der Destillationskolonne liegt dabei vorzugsweise im Bereich von 1 bis 10 bar. Die Destillationskolonne weist vorzugsweise 2 bis 30, insbesondere 5 bis 20 theoretische Trennstufen auf.

Eine weitere Destillation in Verfahrensschritt M dient der weiteren Aufreinigung des Butadiens und kann nach dem Stand der Technik betrieben werden.

Die Erfindung wird durch die nachstehenden Beispiele näher erläutert.

### Beispiel 1

### Herstellung des Pt/Sn/K/Cs/La-Katalysators

Der Katalysator ist ein Pt/Sn/K/Cs/La als aktive Komponenten auf einem ZrO₂/SiO₂-Washcoat enthaltenden Katalysator, wobei der Washcoat auf einen Cordierit-Monolithen aufgebracht ist. Die ZrO₂-Beladung des Monolithen liegt vorzugsweise im Bereich von 2 bis 10 g/inch³.

Ein typische Gehalt, bezogen auf die Gesamtmasse des Katalysators einschließlich des Monolithen, der Aktivkomponenten ist 0,2 Gew.-% Pt, 0,4 Gew.-% Sn, 0,15 Gew.-% K, 0,2 Gew.-% Cs, 2,5 Gew.-% La, 30 Gew.-% Zr und 1,5 Gew.-% Si.

Platin ist dabei das wesentliche Aktivmetall. Si und La stabilisieren den ZrO₂-Träger. K und Cs machen den Katalysator weniger sauer und verringern somit Crack-Reaktionen. Das Zinn stabilisiert unter anderem die Platindispersion.

In der aktiven Form des Katalysators sind Cs, La, K und Si vollständig oxidiert, und Pt ist nicht oxidiert. Das Zinn liegt in einer geringeren Menge als Legierung mit Pt vor, wobei beide metallisch sind, und der größere Anteil des Zinns liegt in oxidischer Form vor.

ZrO₂, stabilisiert mit SiO₂, ist der poröse Träger für die Dopanden K, Cs, La, Sn und Pt. Es wird von ZrO₂-Trägerextrudaten ausgegangen, die 1 Gew.-% eines Polyethylenwachses und 10 bis 15 Gew.-% Stärke als Füllstoff enthalten, bevor die Extrudate kalziniert werden. Die kalzinierten Extrudate werden mit La, K, Cs, Pt und Sn dotiert. Die Füllstoffe werden während des Kalzinierens zu Kohlendioxid und Wasser oxidiert, so dass der fertige Katalysator keine Füllstoffe enthält.

Für die Herstellung des Katalysators kann auf die Beispiele der WO 2010/133565 verwiesen werden, insbesondere Seiten 22 bis 25.

### Beispiel 2

### Sauerstoffentfernung in Stufe H)

Zum Einsatz kam ein Sauerstoff-Entfernungsreaktor einer Miniplant. Der Durchflussreaktor hatte eine Länge von 200 cm, einen äußeren Durchmesser von 0,25 cm, eine Wanddicke von 0,02 cm und einen Innendurchmesser von 0,21 cm. Er war aus Stahl aufgebaut.

Der Reaktor war mit drei externen Heizzonen ausgestattet, die für einen verbesserten Wärmetransfer von den Heizelementen zur Reaktorwand mit Kupferblöcken ausgestattet waren. Um ein adiabatisches System zu erhalten, wurden in der zweiten und dritten Heiz-Zone die Kupferblöcke entfernt und durch Isolationsmaterial ersetzt. In der ersten Aufheiz-Zone wurde eine Vorheiz-Zone eingesetzt, um die Einlassgastemperatur im Reaktor einzustellen. Die zweite und dritte Heiz-Zone wurden so eingestellt, dass Wärmeverluste weitestmöglich verhindert wurden. Der Röhrenreaktor wurde erst hinter dem Ende der ersten Heiz-Zone mit Katalysator befüllt. Ein pneumatisch betriebenes, multiples Thermoelement mit vier Mess-Stellen wurde zur Bestimmung der Temperaturprofile mit einer Auflösung von 2 cm im Katalysatorbett eingesetzt. Das Katalysatorbett wurde zwischen einem Inert-Material (Steatit) gepackt, das als Schutzbett diente. Sowohl isotherme als auch adiabatische Fahrweisen wurden untersucht.

Ein alternativer Reaktor im Labormaßstab wies eine Länge von 70 cm, einen Außendurchmesser von 0,25 cm, eine Wandstärke von 0,02 cm und einen inneren Durchmesser von 0,21 cm auf. Er war aus Stahl aufgebaut.

Typische Reaktionsbedingungen waren ein Katalysatorvolumen von 0,1 I, eine Katalysatormenge von 0,01 bis 0,1 kg, eine GHSV von 2000 bis 10000 NI_{gas}I_{Kat}⁻¹h⁻¹, eine Einlass-Temperatur von 150 bis 410°C und ein Auslassdruck von 1,5 bis 2,5 bara.

Ein typischer Einlassgasstrom enthielt 15 bis 20 Vol.-% C₄-Kohlenwasserstoffe (70 Vol.-% Butadien und 30 Vol.-% Butan), 10 bis 20 Vol.-% Wasser, 5 bis 10 Vol.-% Wasserstoff, 50 bis 60 Vol.-% Stickstoff und 3 bis 5 Vol.-% Sauerstoff.

Bei einer Fahrweise ohne Wasserstoff wurde der Wasserstoff-Anteil durch Inertgas ersetzt.

Ziel des Verfahrens ist die Verringerung des Sauerstoffgehalts auf Werte von weniger als 100 ppm am Reaktorausgang. Für Verfahren ohne Wasserstoffzusatz beziehen sich die Ausbeuten auf CO₂ und Spuren von CO. Im Verfahren mit Wasserstoff-Einsatz beziehen sich die Ausbeuten auf CO₂ und CO, wie auf Dehydrierungsprodukte von Butadien (Buten-Isomere).

Bei einer Temperatur von 410°C, einem Druck von 0,5 bar/g und einer GHSV von etwa 3000 h⁻¹ wurden für den Katalysator aus Beispiel 1 ohne Zusatz von Wasserstoff Restsauerstoff-Gehalte von 100 ppm und bei Mitverwendung von Wasserstoff von unter 100 ppm bestimmt.

Wurde alternativ ein Katalysator eingesetzt, der 28 Gew.-% Kupfer auf Aluminiumoxid enthielt, so wurden 108 ppm Restsauerstoff ohne Wasserstoff-Zusatz und 100 ppm Restsauerstoff mit Wasserstoff-Zusatz bestimmt.

Der erfindungsgemäße Katalysator erfüllte die Anforderungen und zeigte gleichzeitig eine nur sehr geringe Bildung an Nebenprodukten.

## Patentansprüche

1. Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Butene, Butadien, Wasserstoff, gegebenenfalls Wasserdampf, gegebenenfalls Kohlenstoffoxide und gegebenenfalls Inertgase erhalten wird;
G) Einspeisung eines aus dem Gasstrom b gewonnenen Butan, Butene, Butadien enthaltenden Stoffstroms f und eines sauerstoffhaltigen Gases in mindestens eine zweite Dehydrierzone und oxidative Dehydrierung von 1-Buten und 2-Butenen, wobei ein Gasstrom g enthaltend n-Butan, nicht umgesetzte(s) 1-Buten und 2-Butene, Butadien, Wasserdampf, gegebenenfalls Kohlenstoffoxide, gegebenenfalls Wasserstoff und gegebenenfalls Inertgase erhalten wird, und
H) Entfernung des in dem Gasstrom g enthaltenen Restsauerstoffs mittels katalytischer Verbrennungsstufe, in der der Sauerstoff mit einem Teil des oder dem gesamten zuvor abgetrennten Wasserstoff(s) d2 und/oder zusätzlich eingespeistem Wasserstoff umgesetzt wird unter Erhalt eines sauerstoffabgereicherten Stoffstromes h,
**dadurch gekennzeichnet, dass** Stufe H) in Gegenwart eines Katalysators durchgeführt wird, der einen Monolithen umfasst, der ein katalytisch inertes Material mit einer niedrigen BET-Oberfläche von weniger als 10 m²/g und einer Katalysatorschicht aufweist, die auf den Monolithen aufgebracht wurde und ein oxidisches Trägermaterial, mindestens ein Edelmetall ausgewählt aus der Gruppe bestehend aus den Edelmetallen der Gruppe VIII des Periodensystems der Elemente, gegebenenfalls Zinn und/oder Rhenium, und gegebenenfalls weitere Metalle enthält, wobei die Dicke der Katalysatorschicht 5 bis 500 µm beträgt, und das Verfahren zwischen den Stufen B) und G) die nachfolgenden Stufen C) bis F) umfasst, wobei der Stoffstrom f in Stufe G) geführt wird:
C) Kompression in mindestens einer ersten Kompressionsstufe und Abkühlung des Gasstroms b, wobei mindestens ein Kondensatstrom c1 enthaltend Wasser und ein Stoffstrom c2 enthaltend Butene und Butadien, n-Butan, Wasserstoff, Wasserdampf, gegebenenfalls Kohlenstoffoxide und gegebenenfalls Inertgase erhalten wird;
D) Absorption der Butene und des Butadien, n-Butan, Wasserstoff, Wasserdampf, gegebenenfalls Inertgase und gegebenenfalls Kohlenstoffoxide enthaltenden Stoffstroms c2 mit einem 80 bis 97 Gew.-% N-Methylpyrrolidon und 3 bis 20 Gew.-% Wasser enthaltenen Gemisch als selektivem Lösungsmittel, wobei ein N-Methylpyrrolidon, Wasser sowie Butene, Butadien, Butan und gegebenenfalls Kohlendioxid enthaltender Stoffstrom d1 und ein Wasserstoff und gegebenenfalls Inertgase und Butan enthaltender Stoffstrom d2 erhalten werden;
E) Extraktivdestillation des N-Methylpyrrolidon, Wasser sowie Butene, Butadien, Butan und gegebenenfalls Kohlenstoffoxide enthaltenden Stoffstrom d1 mit einem 80 bis 97 Gew.-% N-Methylpyrrolidon und 3 bis 20 Gew.-% Wasser enthaltenden Stoffstrom e1 als selektivem Lösungsmittel, wobei der N-Methylpyrrolidon, Wasser sowie Butene, Butadien, Butan und gegebenenfalls Kohlenstoffoxide enthaltende Stoffstrom d1 in einen N-Methylpyrrolidon, Wasser sowie Butan, Butene, Butadien enthaltenden Stoffstrom e2 sowie einen im Wesentlichen Butan und gegebenenfalls Kohlenstoffoxide enthaltenden Stoffstrom e3 getrennt wird;
F) Destillation des N-Methylpyrrolidon, Wasser, Butan sowie Butene, Butadien enthaltenden Stoffstroms e2 in einen im Wesentlichen N-Methylpyrrolidon und Wasser enthaltenden Stoffstrom e1 und einen Butan, Butene, Butadien enthaltenden Stoffstrom f.

2. Verfahren nach Anspruch 1, **dadurch gekennzeichnet, dass** der Stoffstrom d2 ganz oder teilweise in die erste Dehydierzone B) zurückgeführt wird und/oder der Stoffstrom e1 ganz oder teilweise in die Absorptionsstufe D) und die Extraktivdestillationszone E) zurückgeführt wird, und/oder der Stoffstrom e3 ganz oder teilweise in Stufe A) zurückgeführt wird.

3. Verfahren nach einem der Ansprüche 1 oder 2, **dadurch gekennzeichnet, dass** die Katalysatorschicht in Stufe H) Platin und Zinn umfasst.

4. Verfahren nach einem der Ansprüche 1 bis 3, **dadurch gekennzeichnet, dass** der Katalysator in Stufe H) in der Katalysatorschicht ein Metall aus der dritten Übergangsgruppe des Periodensystems der Elemente einschließlich der Lanthaniden umfasst, vorzugsweise Lanthan.

5. Verfahren nach einem der Ansprüche 1 bis 4, **dadurch gekennzeichnet, dass** der Katalysator in Stufe H) in der Katalysatorschicht ein Alkali- oder Erdalkalimetall umfasst, vorzugsweise Kalium und/oder Cäsium.

6. Verfahren nach einem der Ansprüche 1 bis 5, **dadurch gekennzeichnet, dass** im Katalysator der Stufe H) das oxidische Trägermaterial ausgewählt ist aus Oxiden von Metallen der zweiten, dritten und vierten Hauptgruppe und der dritten und vierten Übergangsgruppe, vorzugsweise Oxiden von Magnesium, Calcium, Aluminium, Silicium, Titan, Zirconium oder Gemischen davon, insbesondere ZrO₂ und SiO₂ oder Gemischen davon.

7. Verfahren nach einem der Ansprüche 1 bis 6, **dadurch gekennzeichnet, dass** im Katalysator der Stufe H) der Monolith Cordierit umfasst.

8. Verfahren nach einem der Ansprüche 1 bis 7, **dadurch gekennzeichnet, dass** in den Stufen B) und H) Katalysatoren mit identischer Zusammensetzung verwendet werden.

9. Verfahren nach einem der Ansprüche 1 bis 8, **dadurch gekennzeichnet, dass** nach Stufe G) oder H) die folgenden Stufen I) bis L) und gegebenenfalls M) durchgeführt werden
I) Kompression in mindestens einer ersten Kompressionsstufe und Abkühlung des sauerstoffabgereicherten Stoffstromes h oder Gasstroms g, wobei mindestens ein Kondensatstrom i1 enthaltend Wasser und ein Gasstrom i2 enthaltend n-Butan, 1-Buten, 2-Butene, Butadien, Wasserstoff, Wasserdampf, gegebenenfalls Kohlenstoffoxide und gegebenenfalls Inertgase erhalten wird;
J) Abtrennung der nicht kondensierbaren und leicht siedenden Gasbestandteile, umfassend Wasserstoff, Sauerstoff, Kohlenstoffoxide, die leicht siedenden Kohlenwasserstoffe Methan, Ethan, Ethen, Propan, Propen und Inertgase als Gasstrom j2 aus dem Gasstrom i2, wobei ein C₄-Produktgasstrom j1 erhalten wird, der im Wesentlichen aus C4-Kohlenwasserstoffen besteht, wobei der Gasstrom j2 ganz oder teilweise in die zweite Dehydrierzone G) zurückgeführt werden kann und die Abtrennung in Stufe J) zweistufig durch Absorption mit nachfolgender Desorption erfolgen kann;
K) Auftrennung des Gasstroms j1 durch Extraktivdestillation mit einem selektiven Lösungsmittel, wie 80 bis 97 Gew.-% N-Methylpyrrolidon und 3 bis 20 Gew.-% Wasser enthaltenden Gemisch in einen Butadien und selektives Lösungsmittel, wie N-Methylpyrrolidon enthaltenden Stoffstrom k1 und einen n-Butan, Butene, Wasserdampf und gegebenenfalls Inertgase enthaltenden Stoffstrom k2 der ganz oder teilweise in den Einsatzstrom in Stufe A), die Absorptionsstufe D), die Extraktionsstufe E) und/oder teilweise die zweite Dehydrierzone G) zurückgeführt werden kann;
L) Destillation des selektiven Lösungsmittels, wie N-Methylpyrrolidon, Wasser und Butadien enthaltenden Stoffstroms k1 in einen im Wesentlichen selektiven Lösungsmittel, wie N-Methylpyrrolidon und Wasser enthaltenden Stoffstrom I1 und einen Butadien enthaltenden Stoffstrom I2, wobei der Stoffstrom I1 ganz oder teilweise in die Stufe K) zurückgeführt werden kann;
M) Reindestillation des Butadien enthaltenen Stoffstromes I2 in einer oder zwei Kolonnen, bei der ein Butadien enthaltender Strom m2 gewonnen wird und ein gegenüber Butadien leichter flüchtige Verunreinigungen enthaltender Gasstrom m1 und/oder ein gegenüber Butadien schwerer flüchtige Verunreinigungen enthaltender Sumpfstrom m3 abgetrennt werden.

10. Verfahren nach einem der Ansprüche 1 bis 9, **dadurch gekennzeichnet, dass** die nicht-oxidative, katalytische Dehydrierung von n-Butan autotherm unter Einspeisung eines sauerstoffhaltigen Gases durchgeführt wird.

11. Verfahren nach Anspruch 10, **dadurch gekennzeichnet, dass** als sauerstoffhaltiges Gas Luft oder mit Sauerstoff angereicherte Luft oder als sauerstoffhaltiges Gas technisch reiner Sauerstoff eingespeist wird.

12. Verfahren nach einer der Ansprüche 1 bis 11, **dadurch gekennzeichnet, dass** der n-Butan enthaltende Einsatzstrom a aus liquefied petroleum gas (LPG) gewonnen wird.

13. Verfahren nach einem der Ansprüche 1 bis 12, **dadurch gekennzeichnet, dass** ein zusätzlicher butenhaltiger Feedstrom in Stufe G) eingespeist wird.

## Claims

1. A process for preparing butadiene from n-butane, which comprises the steps
A) provision of a feed gas stream a comprising n-butane;
B) introduction of the feed gas stream a comprising n-butane into at least one first dehydrogenation zone and nonoxidative catalytic dehydrogenation of n-butane to give a gas stream b comprising n-butane, 1-butene, 2-butenes, butadiene, hydrogen, possibly water vapor, possibly carbon oxides and possibly inert gases;
G) introduction of a stream f which comprises butane, butenes, butadiene and has been obtained from the gas stream b and of an oxygen-comprising gas into at least one second dehydrogenation zone and oxidative dehydrogenation of 1-butene and 2-butenes to give a gas stream g comprising n-butane, unreacted 1-butene and 2-butenes, butadiene, water vapor, possibly carbon oxides, possibly hydrogen and possibly inert gases and
H) removal of the residual oxygen comprised in the gas stream g by means of a catalytic combustion stage in which the oxygen is reacted with part or all of the hydrogen d2 which has previously been separated off and/or additionally introduced hydrogen to give an oxygen-depleted stream h,
wherein step H) is carried out in the presence of a catalyst which comprises a monolith which comprises a catalytically inert material having a low BET surface area of less than 10m²/g and a catalyst layer which has been applied to the monolith and comprises an oxidic support material, at least one noble metal selected from the group consisting of the noble metals of group VIII of the Periodic Table of the Elements, optionally tin and/or rhenium, and optionally further metals, where the thickness of the catalyst layer is from 5 to 500 µm, and the process comprises the following steps C) to F) between steps B) and G), with the stream f being introduced in step G):
C) compression in at least one first compression stage and cooling of the gas stream b, to give at least one condensate stream c1 comprising water and a stream c2 comprising butenes and butadiene, n-butane, hydrogen, water vapor, possibly carbon oxides and possibly inert gases;
D) absorption of the butenes and of the stream c2 comprising butadiene, n-butane, hydrogen, water vapor, possibly inert gases and possibly carbon oxides by means of a mixture comprising from 80 to 97% by weight of N-methylpyrrolidone and from 3 to 20% by weight of water as selective solvent, to give a stream d1 comprising N-methylpyrrolidone, water and butenes, butadiene, butane and possibly carbon dioxide and a stream d2 comprising hydrogen and possibly inert gases and butane;
E) extractive distillation of the stream d1 comprising N-methylpyrrolidone, water and butenes, butadiene, butane and possibly carbon oxides by means of a stream e1 comprising from 80 to 97% by weight of N-methylpyrrolidone and from 3 to 20% by weight of water as selective solvent, with the stream d1 comprising N-methylpyrrolidone, water and butenes, butadiene, butane and possibly carbon oxides being separated into a stream e2 comprising N-methylpyrrolidone, water and butane, butenes, butadiene and a stream e3 comprising essentially butane and possibly carbon oxides;
F) distillation of the stream e2 comprising N-methylpyrrolidone, water, butane and butenes, butadiene to give a stream e1 comprising essentially N-methylpyrrolidone and water and a stream f comprising butane, butenes, butadiene.

2. The process according to claim 1, wherein all or part of the stream d2 is recirculated to the first dehydrogenation zone B) and/or all or part of the stream e1 is recirculated to the absorption step D) and the extractive distillation zone E) and/or all or part of the stream e3 is recirculated to step A).

3. The process according to either of claims 1 and 2, wherein the catalyst layer in step H) comprises platinum and tin.

4. The process according to any of claims 1 to 3, wherein the catalyst layer of the catalyst in step H) comprises a metal of the third transition group of the Periodic Table of the Elements including the lanthanides, preferably lanthanum.

5. The process according to any of claims 1 to 4, wherein the catalyst layer of the catalyst in step H) comprises an alkali metal or alkaline earth metal, preferably potassium and/or cesium.

6. The process according to any of claims 1 to 5, wherein the oxidic support material in the catalyst of step H) is selected from among oxides of metals of the second, third and fourth main groups and the third and fourth transition groups, preferably oxides of magnesium, calcium, aluminum, silicon, titanium, zirconium or mixtures thereof, in particular ZrO₂ and SiO₂ or mixtures thereof.

7. The process according to any of claims 1 to 6, wherein the monolith in the catalyst of step H) comprises cordierite.

8. The process according to any of claims 1 to 7, wherein catalysts having an identical composition are used in steps B) and H).

9. The process according to any of claims 1 to 8, wherein the following steps I) to L) and optionally M) are carried out after step G) or H)
I) compression in at least a first compression stage and cooling of the oxygen-depleted stream h or gas stream g to give at least one condensate stream i1 comprising water and a gas stream i2 comprising n-butane, 1-butene, 2-butenes, butadiene, hydrogen, water vapor, possibly carbon oxides and possibly inert gases;
J) separation of the incondensable and low-boiling gas constituents comprising hydrogen, oxygen, carbon oxides, the low-boiling hydrocarbons methane, ethane, ethene, propane, propene and inert gases as gas stream j2 from the gas stream i2 to give a C₄ product gas stream j1 which consists essentially of C₄-hydrocarbons, with all or part of the gas stream j2 being able to be recirculated to the second dehydrogenation zone G) and the separation in step J) being able to be carried out in two stages by absorption with subsequent desorption;
K) separation of the gas stream j1 by extractive distillation by means of a selective solvent, e.g. a mixture comprising from 80 to 97% by weight of N-methylpyrrolidone and from 3 to 20% by weight of water, into a stream k1 comprising butadiene and selective solvent, such as N-methylpyrrolidone, and a stream k2 comprising n-butane, butenes, water vapor and possibly inert gases which can be recirculated in full or in part to the feed stream in step A), the absorption step D), the extraction step E) and/or in part to the second dehydrogenation zone G);
L) distillation of the selective solvent, e.g. stream k1 comprising N-methylpyrrolidone, water and butadiene to give a stream 11 comprising essentially selective solvents, such as N-methylpyrrolidone and water, and a stream 12 comprising butadiene, with all or part of the stream 11 being able to be recirculated to the step K);
M) pure distillation of the stream 12 comprising butadiene in one or two columns, in which a stream m2 comprising butadiene is obtained and a gas stream m1 comprising impurities which are more volatile than butadiene and/or a bottom stream m3 comprising impurities which are less volatile than butadiene is/are separated off.

10. The process according to any of claims 1 to 9, wherein the nonoxidative catalytic dehydrogenation of n-butane is carried out autothermally with introduction of an oxygen-comprising gas.

11. The process according to claim 10, wherein air or oxygen-enriched air is introduced as oxygen-comprising gas or technical-grade oxygen is introduced as oxygen-comprising gas.

12. The process according to any of claims 1 to 11, wherein the feed stream a comprising n-butane is obtained from liquefied petroleum gas (LPG).

13. The process according to any of claims 1 to 12, wherein an additional feed stream comprising butene is introduced in step G).

## Revendications

1. Procédé pour la préparation de butadiène à partir de n-butane présentant les étapes consistant à A) mettre à disposition un flux gazeux d'utilisation a contenant du n-butane ;
B) injecter le flux gazeux d'utilisation a, contenant du n-butane, dans au moins une première zone de déshydrogénation et déshydrogéner par voie catalytique non oxydante le n-butane, un flux gazeux b, contenant du n-butane, du 1-butène, des 2-butènes, du butadiène, de l'hydrogène, le cas échéant de la vapeur d'eau, le cas échéant des oxydes de carbone et le cas échéant des gaz inertes étant obtenu ;
G) injecter un flux de substances f, contenant du butane, des butènes, du butadiène, obtenu à partir du flux gazeux b, et un gaz contenant de l'oxygène dans au moins une deuxième zone de déshydrogénation et déshydrogéner de manière oxydante le 1-butène et les 2-butènes, un flux gazeux g, contenant du n-butane, du 1-butène et des 2-butènes non transformés, du butadiène, de la vapeur d'eau, le cas échéant des oxydes de carbone, le cas échéant de l'hydrogène et le cas échéant des gaz inertes étant obtenu, et
H) éliminer l'oxygène résiduel contenu dans le flux gazeux g au moyen d'une étape de combustion catalytique, dans laquelle l'oxygène est transformé avec une partie ou la totalité de l'hydrogène d2 séparé au préalable et/ou de l'hydrogène injecté en plus, un flux de substances h appauvri en oxygène étant obtenu,
**caractérisé en ce que** l'étape H) est réalisée en présence d'un catalyseur qui contient un monolithe, qui présente un matériau catalytiquement inerte présentant une surface BET basse, inférieure à 10 m²/g et une couche catalytique, qui a été appliquée sur le monolithe et qui contient un matériau support oxyde, au moins un métal noble choisi dans le groupe constitué par les métaux nobles du groupe VIII du système périodique des éléments, le cas échéant de l'étain et/ou du rhénium et le cas échéant d'autres métaux, l'épaisseur de la couche catalytique étant de 5 à 500 µm, et le procédé comprend, entre les étapes B) et G) les étapes C) à F) suivantes, le flux de substances f étant guidé dans l'étape G) :
C) compression dans au moins une première étape de compression et refroidissement du flux gazeux b, au moins un flux de condensat c1, contenant de l'eau, et un flux de substances c2, contenant des butènes et du butadiène, du n-butane, de l'hydrogène, de la vapeur d'eau, le cas échéant des oxydes de carbone et le cas échéant des gaz inertes étant obtenus ;
D) absorption du flux gazeux c2, contenant des butènes et du butadiène, du n-butane, de l'hydrogène, de la vapeur d'eau, le cas échéant des gaz inertes et le cas échéant des oxydes de carbone, avec un mélange comprenant 80 à 97% en poids de N-méthylpyrrolidone et 3 à 20% en poids d'eau comme solvant sélectif, un flux de substances d1, contenant de la N-méthylpyrrolidone, de l'eau ainsi que des butènes, du butadiène, du butane et le cas échéant du dioxyde de carbone, et un flux de substances d2, contenant de l'hydrogène et le cas échéant des gaz inertes et du butane, étant obtenus ;
E) distillation extractive du flux de substances d1, contenant de la N-méthylpyrrolidone, de l'eau ainsi que des butènes, du butadiène, du butane et le cas échéant des oxydes de carbone, avec un flux de substances e1, contenant 80 à 97% en poids de N-méthylpyrrolidone et 3 à 20% d'eau comme solvant sélectif, le flux de substances d1, contenant de la N-méthylpyrrolidone, de l'eau ainsi que des butènes, du butadiène, du butane et le cas échéant des oxydes de carbone, étant séparé en un flux de substances e2, contenant de la N-méthylpyrrolidone, de l'eau ainsi que du butane, des butènes, du butadiène, ainsi qu'en un flux de substances e3, contenant essentiellement du butane et le cas échéant des oxydes de carbone ;
F) distillation du flux de substances e2, contenant de la N-méthylpyrrolidone, de l'eau, du butane ainsi que des butènes, du butadiène, en un flux de substances e1, contenant essentiellement de la N-méthylpyrrolidone et de l'eau, et en un flux de substances f, contenant du butane, des butènes, du butadiène.

2. Procédé selon la revendication 1, **caractérisé en ce que** le flux de substances d2 est recyclé totalement ou partiellement dans la première zone de déshydrogénation B) et/ou le flux de substances e1 est recyclé totalement ou partiellement dans l'étape d'absorption D) et la zone de distillation extractive E) et/ou le flux de substances e3 est recyclé totalement ou partiellement dans l'étape A).

3. Procédé selon l'une quelconque des revendications 1 ou 2, **caractérisé en ce que** la couche catalytique dans l'étape H) contient du platine et de l'étain.

4. Procédé selon l'une quelconque des revendications 1 à 3, **caractérisé en ce que** le catalyseur dans l'étape H) comprend, dans la couche catalytique, un métal du troisième groupe de transition du système périodique des éléments, y compris des lanthanides, de préférence du lanthane.

5. Procédé selon l'une quelconque des revendications 1 à 4, **caractérisé en ce que** le catalyseur dans l'étape H) comprend, dans la couche catalytique, un métal alcalin ou alcalino-terreux, de préférence du potassium et/ou du césium.

6. Procédé selon l'une quelconque des revendications 1 à 5, **caractérisé en ce que**, dans le catalyseur de l'étape H), le matériau support oxyde est choisi parmi les oxydes de métaux du deuxième, troisième et quatrième groupe principal et du troisième et du quatrième groupe de transition, de préférence les oxydes du magnésium, du calcium, de l'aluminium, du silicium, du titane, du zirconium ou de leurs mélanges, en particulier le ZrO₂ et le SiO₂ ou leurs mélanges.

7. Procédé selon l'une quelconque des revendications 1 à 6, **caractérisé en ce que** le catalyseur dans l'étape H) comprend de la cordiérite monolithique.

8. Procédé selon l'une quelconque des revendications 1 à 7, **caractérisé en ce qu'**on utilise, dans les étapes B) et H), des catalyseurs présentant une composition identique.

9. Procédé selon l'une quelconque des revendications 1 à 8, **caractérisé en ce qu'**après l'étape G) ou H), les étapes I) à L) et le cas échéant M) suivantes sont réalisées :
I) compression dans au moins une première étape de compression et refroidissement du flux de substances h appauvri en oxygène ou du flux gazeux g, au moins un flux de condensat i1, contenant de l'eau, et un flux gazeux i2, contenant du n-butane, du 1-butène, des 2-butènes, du butadiène, de l'hydrogène, de la vapeur d'eau, le cas échéant des oxydes de carbone et le cas échéant des gaz inertes étant obtenus ;
J) séparation des constituants gazeux non condensables et à bas point d'ébullition, comprenant de l'hydrogène, de l'oxygène, des oxydes de carbone, les hydrocarbures à bas point d'ébullition méthane, éthane, éthylène, propane, propène, et des gaz inertes en tant que flux gazeux j2 du flux gazeux i2, un flux gazeux j1 de produits en C4, qui est essentiellement constitué d'hydrocarbures en C4, étant obtenu, le flux gazeux j2 pouvant être recyclé totalement ou partiellement dans la deuxième zone de déshydrogénation G) et la séparation dans l'étape J) pouvant avoir lieu en deux étapes par absorption avec une désorption consécutive ;
K) séparation du flux gazeux j1 par distillation extractive à l'aide d'un solvant sélectif, tel qu'un mélange contenant 80 à 97% en poids de N-méthylpyrrolidone et 3 à 20% en poids d'eau, en un flux de substances k1, contenant du butadiène et du solvant sélectif, tel que de la N-méthylpyrrolidone, et en un flux de substances k2, contenant du n-butane, des butènes, de la vapeur d'eau et le cas échéant des gaz inertes, qui peut être recyclé totalement ou partiellement dans le flux d'utilisation dans l'étape A), l'étape d'absorption D), l'étape d'extraction E) et/ou partiellement dans la deuxième zone de déshydrogénation G) ;
L) distillation du flux de substances k1, contenant du solvant sélectif, tel que de la N-méthylpyrrolidone, de l'eau et du butadiène, en un flux de substances 11, contenant essentiellement du solvant sélectif, tel que de la N-méthylpyrrolidone, et de l'eau, et en un flux de substances 12 contenant du butadiène, le flux de substances 11 pouvant être recyclé totalement ou partiellement dans l'étape K) ;
M) distillation pure du flux de substances 12 contenant du butadiène dans une ou deux colonnes, lors de laquelle on obtient un flux m2 contenant du butadiène et un flux gazeux m1 contenant des impuretés plus volatiles que le butadiène et/ou un flux de fond m3 contenant des impuretés moins volatiles que le butadiène.

10. Procédé selon l'une quelconque des revendications 1 à 9, **caractérisé en ce que** la déshydrogénation catalytique non oxydante du n-butane est réalisée en mode autothermal avec injection d'un gaz contenant de l'oxygène.

11. Procédé selon la revendication 10, **caractérisé en ce qu'**on injecte, en tant que gaz contenant de l'oxygène, de l'air ou de l'air enrichi en oxygène ou, en tant que gaz contenant de l'oxygène, de l'oxygène techniquement pur.

12. Procédé selon l'une quelconque des revendications 1 à 11, **caractérisé en ce que** le flux d'utilisation a contenant du n-butane est obtenu à partir de gaz de pétrole liquéfié (GPL).

13. Procédé selon l'une quelconque des revendications 1 à 12, **caractérisé en ce qu'**on injecte un flux d'alimentation supplémentaire, contenant du butène, dans l'étape G).
